# EUROPEAN PATENT APPLICATION

(11) **EP 3 633 382 A1**
(43) Date of publication of application: **08.04.2020**
(21) Application number: 19199735.2
(22) Date of filing: 03.06.2015
(51) Int. Cl.: G01N 33/68

(54) **NEW MARKERS FOR THE ASSESSMENT OF AN INCREASED RISK FOR MORTALITY**

(30) Priority: 05.06.2014 EP 14305856; 30.09.2014 EP 14306530
(62) Divisional of application: 15728476.1
(71) Applicant: Sanofi-Aventis Deutschland GmbH, 65929 Frankfurt (DE); McMaster University, Hamilton, Ontario L8S 4L8 (CA)
(72) Inventor: HESS, Sybille, 65926 Frankfurt am Main (DE); GERSTEIN, Hertzel, Hamilton, Ontario L8S 4A8 (CA); PARE, Guillaume, Oakville, Ontario L6H 2B5 (CA); MCQUEEN, Matthew, Burlington, Ontario L7P 3A8 (CA)
(74) Representative: Weickmann & Weickmann PartmbB

(57) **Abstract**

The present invention relates to methods for assessing an increased risk for mortality comprising the determination of biochemical markers. It also relates to the use of the biochemical markers or marker panels for the assessment of an increased risk for mortality and to kits for performing the methods of the invention as well as to the therapeutic use of insulin analogues for reducing morality.

## Description

The present invention relates to a method for assessing an increased risk for mortality in a subject as described herein. It discloses the use of at least one marker selected from the group consisting of alpha-Glutathione-S-Transferase, Trefoil Factor 3, alpha-2-Macroglobulin, Macrophage-derived Chemokine, Apolipoprotein B, Selenoprotein P, Tenascin C and Hepatocyte Growth Factor Receptor; and optionally the amount of at least a further marker wherein said further marker is selected from the group consisting of Nt-proBNP, Angiopoietin 2, Growth Differentiation Factor 15, Peroxiredoxin 4, YKL40, Insulin-like Growth Factor Binding Protein 2, Osteoprotegerin and Chromogranin A in the assessment of an increased risk for mortality in a subject as described herein. The invention further pertains to an insulin analogue for use in reducing mortality in a pre-diabetic or diabetic subject as described herein.

Prediction of mortality is important in order to implement suitable therapeutic strategies in order to delay mortality. In particular, cardiovascular (CV) disease is the leading cause of death world-wide.

A number of risk markers for mortality are known:
The B-type natriuretic peptide (BNP) (also termed Brain natriuretic peptide) is a protein with 32 amino acids. It is secreted by the ventricles of the heart in response to excessive stretching of heart muscle cells. BNP is secreted as propeptide along with 76 amino acid long N-terminal fragment (NT-pro BNP; Swiss Prot Number P16860) which is biologically inactive. The biological half life of NT-pro BNP is higher than that of BNP, which makes NT-pro BNP an attractive diagnostic target. Increasing NT-pro BNP plasma levels (together with MCP-1 and Galectin-3) have been found to be associated with a greater incidence of CV events (Tunon J, Am J Cardiol, 2013). Furthermore, NT-pro BNP was identified as predictor of total mortality and CV morality in elderly (Muscari A, Int J Clin Pract, 2013). NT-pro BNP (and hs-cTnT) was also found to improve the accuracy with which the risk of CV events or death can be estimated in patients with type 2 diabetes (Hillis et al. 2013, Diabetes Care 37(1):295-303). NT-pro BNP (+hsTNT) was further found to predict mortality in stable coronary artery disease (Giannitsis E, Clin Chem Lab Med, 2013). Inclusion of NT-pro BNP markedly improved heart failure (HF) risk prediction of Framingham, Health ABC, and ARIC risk scores by 18%, 12%, and 13%. (Agarwal SK, Circ Heart Fail, 2012).

Peroxiredoxin-4 (Uniprot Number Q13162) is a secretable and stable isoform of the peroxiredoxin (Prx) family of antioxidant peroxidases which consists of 6 members. Due to its antioxidant activity, it is a protector against oxidative stress. Peroxiredoxin-4 is involved in the activation of the transcription factor NF-kappaB. It has been shown that elevated serum level are associated with a significant higher risk of incident cardiovascular events and cardiovascular mortality and all-cause mortality in patients without history of cardiovascular disease (Abbasi A, JAHA, 2012). Moreover, high levels of Peroxiredoxin-4 have been observed in patients with type 2 diabetes and peripheral atherosclerosis disease (PAD) (Eter El, Cell Stress Chaperones, 2013).

Angiopoietin-2 (Ang-2; SwissProt Number 015123) is a protein that is encoded by the ANGPT2 gene in humans. ANG-2 belongs to the angiopoietin family which comprises 4 vascular growth factors that play a role in angiogenesis. Angiopoietin cytokines are involved with controlling microvascular permeability and allow the smooth muscle cells to cover the vessels making vasodilation and vasoconstriction possible. Angiopoietin-2 promotes cell death and disrupts vascularisation. Circulating Ang-2 (and its receptor sTie-2) were determined has heritable traits and were associated with cardiovascular disease risk factors, including metabolic syndrome (Lieb W, Circ CV Genet, 2010). High levels of ANG-2 were found to be associated with a greater risk of all-cause and cardiovascular mortality (Lorbeer R, Eur J Heart Fail, 2013).

According to the SwissProt database, GDF-15 (SwissProt Number Q99988) belongs to the TGF beta superfamily and has a role in inflammatory and apoptotic pathways. Serum GDF-15 has been positively correlated with progression of several tumor types, including certain colorectal, pancreatic, and prostate cancers. GDF-15 is also upregulated by cardiovascular events triggering oxidative stress, including atherosclerosis. Increased circulating GDF-15 concentrations have been linked to an enhanced risk of future adverse cardiovascular events.

YKL-40 (SwissProt number P36222), also known as Chitinase-3-like protein 1, is a carbohydrate-binding lectin with a preference for chitin. It plays a role in defense against pathogens and in tissue remodeling. YKL-40 is thought to play an important role in the capacity of cells to respond to and cope with changes in their environment. It is a potent growth factor for connective tissue cells and acts as a migration factor for endothelial cells. YKL-40 is present in activated macrophages, articular chondrocytes, synovial cells, and the liver. It is undetectable in muscle tissues, lung, pancreas, mononuclear cells, or fibroblasts. YKL-40 binds chitins; however, it has no chitinase activity. Elevated serum levels are seen in arthritis, severe bacterial infection, inflammatory bowel disease, and various cancers (SwissProt database). YKL-40 as predictor of all-cause and cardiovascular mortality has been reported (Rathcke et al., 2010, International Journal of Cardiology, 143:35-42).

Insulin like growth factor binding protein 2 (SwissProt number P18065) (IGFBP-2) is a cysteine-rich protein with conserved cysteine residues clustered in the amino- and carboxy-terminal thirds of the molecule. IGFBPs modulate the biological activities of IGF proteins. Some IGFBPs may also have intrinsic bioactivity that is independent of their ability to bind IGF proteins. During development, IGFBP-2 is expressed in a number of tissues. The highest expression level is found in the central nervous system. In adults, high expression levels are detected in the central nervous system and in a number of reproductive tissues. IGFBP-2 binds preferentially to IGF II, exhibiting a 2 - 10 fold higher affinity for IGF II than for IGF I (SwissProt database). IGFBP-2 was reported to be related and to increase 8-year mortality in elderly men (van den Beld et al., 2012, European Journal of Endocomidology, 167: 111-117).

Chromogranin-A (CgA, SwissProt number P10645) is the major soluble protein co-stored and co-released from neurons and neuroendocrine cells together with catecholamines and can function as a pro-hormone by giving rise to several bioactive peptides including vasostatin, pancreastatin, catestatin, parastatin, chromostatin, WE-14 and GE-25. It is also stored in atrial granules in the myocardium.

CgA and its fragments exert a broad spectrum of regulatory activities by influencing the endocrine, the cardiovascular, and the immune systems and by affecting the glucose or calcium homeostasis.

CgA plasma concentrations associated with all-cause mortality. After adjustment for known risk factors of mortality the association was lost (Rosjo H, Eur J Heart Fail, 2010).

CgA is an independent predictor of long-term mortality and heart failure hospitalizations across the spectrum of ACSs and provides incremental prognostic information to conventional cardiovascular risk markers (Jansson AM, Eur Heart J, 2009).

CgA can identify subjects with increased risk of short- and long-term mortality (Goetze JP, Endocrine Connections, 2014).

Increased CgA (and CT-proET-1) level in subjects with acute destabilized heart failure in the emergency department add independent prognostic information in addition to NT-proBNP measurement (Dieplinger B., Clin Chim Acta, 2009).

Osteoprotegerin (OPG, SwissProt Number O00300) is a cytokine receptor, and a member of the tumor necrosis factor (TNF) receptor superfamily.

OPG mainly binds to 2 ligands:
TRAIL (tumor necrosis factor-related apoptosis-induced ligand)
   - apoptosis of tumor cells prevented.
RANKL (receptor activator of nuclear factor kappa B ligand
   - apoptosis of osteoclasts.
      Regulation of mineral metabolism in bone
      and vascular tissues

OPG can be considered as a "vascular calcification" marker.

Independent predictor of combined end-point of hospitalization of ischaemic heart disease, ischaemic stroke and all-cause mortality (Mogelvang R, Heart, 2012).

Elevated levels are associated with long-term renal dysfunction (Lewis JR, Am J Nephrol, 2014).

It was the object of the present invention to investigate whether a novel biochemical marker can be identified which may be used in assessing an increased risk for mortality, particularly cardiovascular mortality.

Surprisingly, it has been found that the biochemical markers alpha-Glutathione-S-Transferase (Swiss Prot Number P08263), Trefoil Factor 3 (Swiss Prot Number Q07654), alpha-2-Macroglobulin (Swiss Prot Number P01023), and Macrophage-derived Chemokine (Swiss Prot Number O00626are significant predictors alone, in combination with each other or in combination with known biochemical markers as described herein, particularly when added to one or more of the risk factors of a subject as described herein for the assessment of an increased risk for mortality, particularly within about seven years.

Trefoil Factor 3 (TFF3; Swiss Prot number Q07654) is a protein that in humans is encoded by the TFF3 gene (chromosome 21). Members of the Trefoil family are characterized by having at least one copy of the Trefoil motif, a 40-AA domain that contains three conserved SS-bonds. Trefoil factors are secretory products of mucin producing cells. They play a key role in the maintenance of the surface integrity of oral mucosa and enhance healing of the gastrointestinal mucosa. TFF comprises the gastric peptides (TFF1), spasmolytic peptide (TFF2), and the intestinal Trefoil factor (TFF3). TFF3 has been identified to predict all-cause mortality in urinary samples in subjects with an increased risk of kidney disease (O'Seaghdha et al., 2013, J. Am. Soc. Nephrol., 24: 1880-1888). The cohort (Framingham Heart Study) described by O'Seaghdha et al (table 1) consist of < 10% diabetics; The ORIGIN cohort in contrast consists of > 80 % diabetics.

Alpha-2-Macroglobulin (Swiss Prot Number P01023) is a large plasma protein found in the blood and is produced by the liver. It acts as an antiprotease and is able to inactivate an enormous variety of proteinases. It is an acute-phase protein. It functions also as a carrier of cytokines, growth factors and hormones. Alpha-2-Macroglobulin rises 10-fold or more in serum in the nephrotic syndrome. The loss of alpha-2-Macroglobulin into urine is prevented by its large size. Alpha-2-Macroglobulin has not been reported to be predictor of all-cause or cardiovascular mortality.

The human alpha class Glutathione-S-Transferases (GSTs) consist of 5 genes, hGSTA1-hGSTA5, and 7 pseudogenes on chromosome 6. Glutathione-S-Transferases (GSTs) comprise a family of eukaryotic and prokaryotic phase II metabolic isozymes best known for their ability to catalyze the conjugation of the reduced form of glutathione (GSH) to xenobiotic substrates for the purpose of detoxification. The mammalian GSTs active in drug metabolism are now classified into the alpha, mu and pi classes Alpha-Glutathione-S-Transferase (Swiss Prot Number P08263) has not been reported to be a predictor of all-cause or cardiovascular mortality.

Macrophage-Derived Chemokine (SwissProt number O00626) (MDC; CCL22) is expressed highly in macrophages and in monocyte-derived dendritic cells. High expression is detected in normal thymus and lower expression in lung and spleen. MDC is expressed by a subset of macrophages within regions of advanced atherosclerotic plaques that contain plaque micro-vessels. MDC is a potent chemoattractant for neutrophilic granulocytes, enhancing their bactericidal activity and stimulating the release of lysozyme (SwissProt database). Macrophage-derived chemokine has not been reported to be a predictor of all-cause or cardiovascular mortality.

Apolipoprotein B (ApoB, SwissProt number P04114) is the primary apolipoprotein of chylomicrons and low-density lipoproteins (LDL).

ApoB on the LDL particle acts as a ligand for LDL receptors in various cells throughout the body.

High levels of ApoB can lead to plaques that cause vascular disease (atherosclerosis), leading to heart disease.

There is considerable evidence that levels of ApoB are a better indicator of heart disease risk than total cholesterol or LDL.

ApoB/ApoA1 ratio superior to any of the cholesterol ratios for estimation of the risk of acute myocardial infarction (McQueen MJ, Lancet,2008).

Apo B (and the Apo B/Apo A-I ratio) are associated with carotid intima-media thickness-(Huang F, PLOSONE, 2013).

Selenoprotein P (SeP, SwissProt number P49908) is the most common selenoprotein found in the plasma (5-6 mg/ml). The liver produces 75% of the protein found in the circulation, but also almost all tissues express the protein.

SeP transports selenium from the liver to extra-hepatic tissues and also has anti-oxidant properties.

SeP elevated in patients with glucose metabolism dysregulation and related to various cardio-metabolic parameters including insulin resistance, inflammation, and atherosclerosis (Yang SL, J Clin Endocrinol Metab, 2011).

Overproduction of SeP is connected
with hypoadiponectinemia in patients with type 2 diabetes (Misu H, PLoSONE, 2012).

Tenascin-C (SwissProt number P24821) belongs to the tenascins. Tenascins are extracellular hexameric matrix glycoproteins. They are pleiotropic regulators of a variety of cell functions associated with embryogenesis, wound healing, cell proliferation, differentiation, motility, and nerve regeneration
There are four members : tenascin-R, tenascin-X and tenascin-W and tenascin-C.

Tenascin-C can be considered as a marker of tissue remodelling and myocardial disease activity.
Serum TN-C (+ BNP level) are independent predictors for cardiac events (Sato A, J Card Fail, 2012).

High serum level of tenascin-C (as well as MMP-9, TIMP_1) associated with decreased survival in subjects with dilated cardiomyopathy (DCM) (Franz M, Int J Cardiol, 2013).

High serum TN-C levels are present in adult ventricular noncompaction/hypertrabeculation (NC/HT), a rare form of congenital cardiomyopathy (Erer HB, Echocardiogr. 2014).

Review: tenascin C in human cardiac pathology. Niebroj-Dobosz I, Clin Chim Acta, 2012.

Hepatocyte Growth Factor Receptor (HGFR, SwissProt number P08581) is a heterodimeric membrane receptor of mesenchymal origin. Upon stimulation, it has mitogenic, antiapoptotic and angiogenic properties with effects on various cell types. It plays a role in embryonic development and wound healing.
Hepatocyte growth factor (HGF) is the only known ligand of HGFR.

High level of HGFR (and its ligand HGF) may reflect attempts to repair failures in organ systems.

Subjects with high level of the ligand, HGF, had more CV disease and higher mortality (Kämppä N, Exp Gerontol, 2013).

Serum levels of the ligand, HGF, correlate with CHF severity and are associated with CV mortality (Lamblin N, Circulation, 2005).

The ligand, HGF, is high in serum of bypass surgery patients with ischemic cardiomyopathy and is a mediator of cardiac stem cells migration (D"Amario, D, Circulation, 2014).

In particular, the inventors have used a randomized pool of patients from the ORIGIN trial and identified in total 10 biochemical markers, i.e. Nt-pro BNP, alpha-Glutathione-S-Transferase, Growth Differentiation Factor 15, Trefoil Factor 3, alpha-2-Macroglobulin, Macrophage-derived Chemokine, Angiopoietin-2, YKL-40, Peroxiredoxin-4 and Insulin-like Growth Factor Binding Protein 2 which alone or in combination with each other or with further biomarkers significantly correlate with mortality, particularly cardiovascular mortality such as fatal myocardial infarction, fatal stroke and/or heart failure.

In addition, for final validation, the forward selection process was repeated with the full 8401 participants for the mortality outcome. The inventors confirmed the biomarkes alpha-Glutathione-S-Transferase, Trefoil Factor 3, alpha-2-Macroglobulin, and Macrophage-derived Chemokine and additionally found the biomarkers Apolipoprotein B, Selenoprotein P, Tenascin-C and Hepatocyte Growth Factor Receptor, which alone or in combination with each other or with further known biomarkers, significantly correlate with mortality, particularly cardiovascular mortality such as as fatal myocardial infarction, fatal stroke and/or heart failure.

In addition, in a modified model in which age and both age and creatinine are added to the basic clinical model, the inventors confirmed alpha- Glutathione-S-Transferase, Trefoil Factor 3, alpha-2-Macroglobulin, Macrophage-derived Chemokine and further identified Selenoprotein P and Tenascin C as biomarkers which alone or in combination with each other or with further known biomarkers significantly correlate with mortality, particularly cardiovascular mortality such as fatal myocardial infarction, fatal stroke and/or heart failure.

The present invention therefore relates to a method, e.g. an in vitro method for assessing an increased risk for mortality, particularly cardiovascular mortality in a subject comprising:
(a) determining in a sample from said subject
   (i) the amount of at least one first marker selected from the group consisting of alpha-Glutathione-S-Transferase, Trefoil Factor 3, alpha-2-Macroglobulin, Macrophage-derived Chemokine, Apolipoprotein B, Selenoprotein P, Tenascin C and Hepatocyte Growth Factor Receptor; and
   (ii) optionally the amount of at least one further marker; and
(b) correlating that said subject is at increased risk for mortality when said amount is altered compared to a reference amount for the at least one first marker.

In a preferred embodiment the further marker is selected from the group consisting of Nt-pro BNP, Angiopoietin-2, Growth Differentiation Factor 15, Peroxiredoxin-4, YKL-40, Insulin-like Growth Factor Binding Protein 2, Osteoprotegerin and Chromogranin A.

In a preferred embodiment of the invention the further marker in the method as described herein is selected from the group consisting of Growth Differentiation Factor 15, Insulin-like Growth Factor Binding Protein 2, Angiopoietin-2, Nt-ProBNP, YKL-40, Osteoprotegerin and Chromogranin A.

"Mortality" in the sense of the present invention is all-cause mortality, for example mortality that is caused by a pathological state or disorder, accident, infection, or suicide. Particularly preferred is mortality caused by a pathological state or disorder. Most preferred is cardiovascular mortality such as fatal myocardial infarction, fatal stroke or heart failure.

A "further marker" in the sense of the present invention is any marker that if combined with the first marker adds relevant information in the assessment of an increased risk for mortality. The information is considered relevant if the relative risk of the further marker is similar to the relative risk of the first marker as defined, e.g., by the hazard ratio.

In one embodiment, the present invention relates to a method, e.g., an in vitro method, for assessing an increased risk for mortality, particularly cardiovascular mortality in a subject, comprising:
(a) determining in a sample from said subject
   (i) the amount of at least one first marker selected from the group consisting of alpha-Glutathione-S-Transferase, Trefoil Factor 3, alpha-2-Macroglobulin, and Macrophage-derived Chemokine; and
   (ii) optionally the amount of at least a further marker; and
(b) correlating that said subject is at increased risk for mortality when said amount is altered compared to a reference amount for the at least one first marker.

In a preferred embodiment of the invention, the further marker in the method as described herein is selected from the group consisting of Nt-pro BNP, Angiopoietin-2, Growth Differentiation Factor 15, Peroxiredoxin-4, YKL-40 and Insulin-like Growth Factor Binding Protein 2.

In a further embodiment, the present invention relates to a method, e.g. an in vitro method for assessing an increased risk for mortality, particularly cardiovascular mortality in a subject comprising:
(a) determining in a sample from said subject
   (i) the amount of at least one first marker selected from the group consisting of alpha-Glutathione-S-Transferase, Trefoil Factor 3, Macrophage-derived Chemokine, alpha-2-Macroglobulin, Selenoprotein P and Tenascin C; and
   (ii) optionally the amount of at least a further marker; and
(b) correlating that said subject is at increased risk for mortality when said amount is altered compared to a reference amount for the at least one marker.

In a preferred embodiment of the invention the further marker in the method as described herein is selected from the group consisting of Growth Differentiation Factor 15, Insulin-like Growth Factor Binding Protein 2, Angiopoietin-2, Nt-ProBNP, YKL-40, and Chromogranin A.

In a further preferred embodiment of the invention, the first marker as described herein is alpha-Glutathione-S-Transferase, optionally with at least a further marker, e.g., wherein said at least further marker is selected from the group consisting of Nt-pro BNP, Angiopoietin-2, Growth Differentiation Factor 15, Peroxiredoxin-4, YKL-40 and Insulin-like Growth Factor Binding Protein 2.

In a particularly preferred embodiment of the invention, the first marker as described herein is alpha-Glutathione-S-Transferase and a further marker Nt-pro BNP and Angiopoietin-2.

In a particularly preferred embodiment of the invention, the first marker as described herein is Hepatocyte Growth Factor Receptor and a further marker Chromogranin A.

In a further preferred embodiment of the invention, the first marker as described herein is Trefoil Factor 3, optionally with at least a further marker, e.g., wherein said at least further marker is selected from the group consisting of Nt-pro BNP, Angiopoietin-2, Growth Differentiation Factor 15,Peroxiredoxin-4, YKL-40 and Insulin-like Growth Factor Binding Protein 2.

In a further preferred embodiment of the invention, the first marker as described herein is alpha-2-Macroglobulin, optionally with at least a further marker, e.g., wherein said at least further marker is selected from the group consisting of Nt-pro BNP, Angiopoietin-2, Growth Differentiation Factor 15, Peroxiredoxin-4, YKL-40 and Insulin-like Growth Factor Binding Protein 2.

In a further preferred embodiment of the invention, the first marker as described herein is Macrophage-derived Chemokine, optionally with at least a further marker, e.g., wherein said at least further marker is selected from the group consisting of Nt-pro BNP, Angiopoietin-2, Growth Differentiation Factor 15, Peroxiredoxin-4, YKL-40 and Insulin-like Growth Factor Binding Protein 2.

In a further embodiment of the invention, the first marker as described herein is selected from the group consisting of alpha-Glutathione-S-Transferase, alpha-2-Macroglobulin and Macrophage-derived Chemokine, optionally with at least a further marker, e.g., wherein said at least further marker is selected from the group consisting of Nt-pro BNP, Angiopoietin-2, Growth Differentiation Factor 15, Peroxiredoxin-4, YKL-40 and Insulin-like Growth Factor Binding Protein 2.

In a further embodiment of the invention, the first marker as described herein is alpha-Glutathione-S-Transferase and alpha-2-Macroglobulin, optionally with at least a further marker, e.g., wherein said at least further marker is selected from the group consisting of Nt-pro BNP, Angiopoietin-2, Growth Differentiation Factor 15, Peroxiredoxin-4, YKL-40 and Insulin-like Growth Factor Binding Protein 2.

In a further embodiment of the invention, the first marker as described herein is alpha-Glutathione-S-Transferase and Macrophage-derived Chemokine, optionally with at least a further marker, e.g., wherein said at least further marker is selected from the group consisting of Nt-pro BNP, Angiopoietin-2, Growth Differentiation Factor 15, Peroxiredoxin-4, YKL-40 and Insulin-like Growth Factor Binding Protein 2.

In a further embodiment of the invention, the first marker as described herein is alpha-2-Macroglobulin and Macrophage-derived Chemokine, optionally with at least a further marker, e.g., wherein said at least further marker is selected from the group consisting of Nt-pro BNP, Angiopoietin-2, Growth Differentiation Factor 15, Peroxiredoxin-4, YKL-40 and Insulin-like Growth Factor Binding Protein 2.

In a further embodiment of the invention, the first marker as described herein is selected from the group consisting of alpha-Glutathione-S-Transferase, alpha-2-Macroglobulin, Macrophage-derived Chemokine, Apolipoprotein B, Selenoprotein P, Tenascin C and Hepatocyte Growth Factor Receptor, optionally with at least a further marker as described herein.

In a further embodiment of the invention, the first marker as described herein is selected from the group consisting of alpha-Glutathione-S-Transferase, Macrophage-derived Chemokine, alpha-2-Macroglobulin, Selenoprotein P and Tenascin C, optionally with at least a further marker as described herein.

In a further embodiment of the invention, the first and further marker as described herein is Nt-pro BNP, alpha-Glutathione-S-Transferase, Growth Differentiation Factor 15, Trefoil Factor 3, alpha-2-Macroglobulin, Macrophage-derived Chemokine, Angiopoietin-2, YKL-40, Peroxiredoxin-4 and Insulin-like Growth Factor Binding Protein 2.

In a further preferred embodiment of the invention the first marker as described herein is Apolipoprotein B, optionally with at least a further marker as described herein.

In a further preferred embodiment of the invention the first marker as described herein is Selenoprotein P, optionally with at least a further marker as described herein.

In a further preferred embodiment of the invention the first marker as described herein is Tenascin C, optionally with at least a further marker as described herein.

In a further preferred embodiment of the invention the first marker as described herein is Hepatocyte Growth Factor Receptor, optionally with at least a further marker as described herein.

In a further embodiment of the invention, the first and further marker as described herein is alpha-Glutathione-S-Transferase, Trefoil Factor 3, alpha-2-Macroglobulin, Macrophage-derived Chemokine, Chromogranin A, Selenoprotein P, Tenascin C, Hepatocyte Growth Factor Receptor, Osteoprotegerin, Apolipoprotein B, Growth-Differentiation-Factor-15, Insulin-like Growth Factor Binding Protein 2, Angiopoietin-2, Nt-proBNP, and YKL-40.

In a further embodiment of the invention, the first and further marker as described herein is alpha-Glutathione-S-Transferase, Trefoil 3, alpha-2-Macroglobulin, Macrophage-derived Chemokine, Apolipoprotein B, Selenoprotein P, Tenascin C, Hepatocyte Growth Factor Receptor, Nt-proBNP, Angiopoietin 2, Growth Differentiation Factor 15, Peroxiredoxin 4, YKL40, Insulin-like Growth Factor Binding Protein 2, Osteoprotegerin and Chomogranin A.

In a further embodiment of the invention, the first and further marker as described herein is alpha-Glutathione-S-Transferase, Trefoil Factor 3, Macrophage-derived Chemokine, alpha-2-Macroglobulin, Chromogranin A, Selenoprotein P, Tenascin C, Growth-Differentiation-Factor-15, Insulin-like Growth Factor Binding Protein 2, Angiopoietin-2, Nt-pro BNP, and YKL-40.

The method according to the present invention as described herein might be used for assessing an increased risk for mortality in a subject wherein said increased risk is within the next 1, 2, 3, 4, 5, 6 or 7 years, particularly within the next 1-7, 1-3, 2-7, 2-5, 3-7, 4-7, or 5-7 years.

In a preferred embodiment the method according to the present invention is used for assessing an increased risk for mortality which is a cardiovascular mortality such as fatal myocardial infarction, fatal stroke, and/or heart failure in a subject.

In a further embodiment of the method and uses of the invention, the first marker is Trefoil Factor 3 and the sample is serum.

In a further preferred embodiment of the method and uses of the invention, the first marker is Trefoil Factor 3 and the mortality is cardiovascular mortality.

In another embodiment of the invention, the first marker is Trefoil Factor 3 and the subject suffers from one or more risk factors as described herein, e.g. the risk factors selected from the group consisting of a previous cardiovascular disorder, albuminuria, male, age of at least 50 years, smoker, diabetic or pre-diabetic, elevated blood cholesterol levels, obesity and hypertension.

In another embodiment of the methods and uses of the invention, the first marker is Trefoil Factor 3, the sample is serum, the mortality is cardiovascular mortality and the subject suffers from one or more risk factors as described herein, e.g. the risk factors selected from the group consisting of a previous cardiovascular disorder, albuminuria, male, age of at least 50 years, smoker, diabetic or pre-diabetic, elevated blood cholesterol levels, obesity and hypertension.

In the method as described herein, the subject might be a subject without a risk of a cardiovascular disorder, such as a subject not fulfilling one or more of the risk factors as defined in a heart study investigating risk factors, e.g. the Interheart Study (Yusuf et al., 2004, Lancet 364:953-962) or the Framingham Heart Study.

Preferably, in the method as described herein, the subject is known to be at higher than average risk of a cardiovascular disorder, such as a subject fulfilling one or more of the risk factors as defined in a heart study investigating risk factors, e.g. the Interheart Study (Yusuf et al., 2004, Lancet 364:953-962) or the Framingham Heart Study. Such a subject might suffer from one or more of the risk factors selected from the group consisting of a previouscardiovascular disorder, albuminuria, male, at least 50 years of age, elevated blood cholesterol levels (e.g. LDL levels above 100 mg/dl (2.5 mmol/L), elevated Creatinine levels (e.g. ≥ 1.0 mg/dl for females and ≥ 1.2 mg/dl for males), obesity, preferably abdominal obesity, smoker, diabetic, e.g., type 1, preferably LADA or type 2 diabetes, high alcohol consumption and/or hypertension (e.g. values above 140 and/or 90 mmHg). In a particularly preferred embodiment, the subject suffer from one or more of the risk factors selected from the group consisting of a previous cardiovascular disorder, albuminuria, male, at least 55 years of age, elevated blood cholesterol levels, smoker, diabetic, e.g., type 1, preferably LADA or type 2 diabetes and/or hypertension. Most preferred is a subject who suffers from the risk factors male, at least 55 years of age and smoker.

In a preferred embodiment the method according to the present invention is used for assessing an increased risk for mortality in a subject, preferably cardiovascular mortality, which had a previous cardiovascular disorder, is pre-diabetic or diabetic, preferably LADA or type 2 diabetes, or has an age of at least 50, 55, 60, 63, or 65 years, preferably 63 years.

A preferred embodiment relates to the method according to the present invention that is used for assessing an increased risk for mortality in a subject which had a previous cardiovascular disorder and is pre-diabetic or diabetic, preferably type 2 diabetic or LADA, and has an age of at least 50, 55, 60, 63, or 65 years, preferably 63 years.

The subject might be a human or non-human animal, such as monkey, rabbit, rat or mouse.

The term "pre-diabetic" or "pre-diabetes" as used throughout the application refers, e.g., to a patient with impaired glucose tolerance (IGT), defined as a PPG value ≥140 and <200 mg/dL (ie, ≥7.8 and <11.1 mmol/L), with a FPG <126 mg/dL (7.0 mmol/L) as determined by an oral glucose tolerance test (OGTT) or a patient with impaired fasting glucose (IFG), defined as an FPG ≥110 and <126 mg/dL (≥6.1 and <7 mmol/L), without diabetes mellitus (PPG must be <200 mg/dL [11.1 mmol/L]) both as determined by, e.g., a 75 g oral glucose tolerance test (OGTT) which is known in the art. For example, an (OGTT) is performed fasting (ie, no consumption of food or beverage other than water for at least 8 hours). Two plasma glucose values are drawn during the OGTT - a fasting value (FPG) and a value drawn two hours after the 75 g oral glucose load was administered (postprandial plasma glucose [PPG]). It also refers to a patient with early type 2 diabetes, defined as a FPG ≥126 mg/dL (7.0 mmol/L) or a PPG of ≥200 mg/dL (11.1 mmol/L).

The term "diabetic" as used throughout the application refers to a subject with type 2 diabetes. The term also refers to a subject with type 1 diabetes, preferably with latent autoimmune diabetes (LADA), e.g., as diagnosed by measuring of autoantibodies against, e.g., GAD as described, e.g., in Naik et al., 2009, J Clin Endocrinol Metab, 94:4635-4644.

The term "cardiovascular disorder" as used throughout the application refers to non-fatal myocardial infarction, non-fatal stroke, non-fatal heart failure, revascularization, e.g., of coronary, carotid or peripheral artery, angina pectoris, left ventricular hypertrophy, stenosis, e.g., of coronary, carotid, or lower extremity arteries.

The term "previous cardiovascular disorder" as used throughout the application refers to a patient with the diagnosis of one or more of the following disorders: non-fatal myocardial infarction (MI); non-fatal stroke; coronary, carotid or peripheral arterial revascularization; angina with documented ischemic changes (at least 2 mm ST segment depression on electrocardiogram during a Graded Exercise Test [GXT]; or with a cardiac imaging study positive for ischemia); or unstable angina with documented ischemic changes (either ST segment depression of at least 1 mm or an increase in troponin above the normal range but below the range diagnostic for acute myocardial infarction); microalbuminuria or clinical albuminuria (an albumin: creatinine ratio ≥ 30 µg/mg in at least one or timed collection of urine with albumin excretion ≥20 µg/min or ≥ 30 mg/24 hours or total protein excretion ≥500 mg/24 hours); left ventricular hypertrophy by electrocardiogram or echocardiogram; significant stenosis on angiography of coronary, carotid, or lower extremity arteries (ie, 50% or more stenosis); and/or ankle-brachial index < 0.9.

The method for assessing an increased risk for mortality as described herein comprises determining in a sample the amount, e.g. presence, level and/or concentration of at least a first marker as described herein and (ii) optionally the amount of at least a further marker as described herein; and (b) correlating that said subject is at an increased risk for mortality when said amount is altered compared to a reference amount for the at least first marker.

The term "determining" comprises a qualitative determination of the amount of said first or further marker as described herein in a sample compared to a reference amount. In a preferred embodiment the determination is a qualitative or semi-quantitative determination, i.e. it is determined whether the concentration of said first or further marker as described herein is above or below a cut off value. As the skilled artisan will appreciate, in a Yes- (presence) or No- (absence) assay, the assay sensitivity is usually set to match the cut-off value. A cut-off value can for example be determined from the testing of a control population. The control population might be a population of randomized subjects regarding e.g., sex, age, risk factors for mortality, such as cardiovascular risk factors as described herein, e.g. smoking, hypertonia, obesity, elevated blood cholesterol levels, pre-diabetes, diabetes and/or increased alcohol consumption. Preferably, the cut-off is set to result in a specificity of 90%, also preferred the cut-off is set to result in a specificity of 95%, or also preferred the cut-off is set to result in a specificity of 98%. Presence of a value above the cut-off value can for example be indicative for the presence of an increased risk for mortality. Alternatively, it is determined whether the concentration of said first or further marker as described herein is within a specific predefined concentration range of said marker and correlated whether the specific predefined range is associated with an increased risk for mortality, e.g. by applying unadjusted and adjusted Cox regression models. For example, the marker concentration range which is found within the whole population is predefined in categories, e.g. 3 categories (tertiles), 4 categories (quartiles) or 5 categories (quintiles), preferably quintiles, with category 1 defining the lowest concentration sub-range and category 5 defining the highest concentration sub-range. The risk for mortality e.g. increases from category 1 to 3-5, respectively.

Alternatively, the term "determining" comprises a quantitative determination of the amount of said first marker as described herein. In this embodiment, the concentration of the marker as described herein is correlated to an underlying diagnostic question like, e.g., classification of pre-mortality stages, follow-up after a previous disorder, e.g. cardiovascular disorder or response to therapy.

As obvious to the skilled artisan, the present invention shall not be construed to be limited to the full-length protein of the first or further marker as described herein. Physiological or artificial fragments of the first or further marker as described herein, secondary modifications of the first or further marker as described herein, as well as allelic variants of the first or further marker as described herein are also encompassed by the present invention. Artificial fragments preferably encompass a peptide produced synthetically or by recombinant techniques, which at least comprises one epitope of diagnostic interest consisting of at least 6 contiguous amino acids as derived from the first or further marker as described herein. Such fragment may advantageously be used for generation of antibodies or as a standard in an immunoassay. More preferred the artificial fragment comprises at least two epitopes of interest appropriate for setting up a sandwich immunoassay.

The articles "a" and "an" are used herein to refer to one or to more than one (i.e. to at least one) of the grammatical object of the article. By way of example, "a marker" means one marker or more than one marker. The term "at least" is used to indicate that optionally one or more further objects may be present. By way of example, a marker panel comprising as markers at least the first marker alpha-Glutathione-S-Transferase and Trefoil Factor 3 and optionally at least one or more further markers.

The term "marker" or "biochemical marker" as used herein refers to a molecule to be used as a target for analyzing a patient's test sample. In one embodiment examples of such molecular targets are proteins or polypeptides. Proteins or polypeptides used as a marker in the present invention are contemplated to include naturally occurring variants of said protein as well as fragments of said protein or said variant, in particular, immunologically detectable fragments. Immunologically detectable fragments preferably comprise at least 6, 7, 8, 10, 12, 15 or 20 contiguous amino acids of said marker polypeptide. One of skill in the art would recognize that proteins which are released by cells or present in the extracellular matrix may be damaged, e.g., during inflammation, and could become degraded or cleaved into such fragments. Certain markers are synthesized in an inactive form, which may be subsequently activated by proteolysis. As the skilled artisan will appreciate, proteins or fragments thereof may also be present as part of a complex. Such complex also may be used as a marker in the sense of the present invention. Variants of a marker polypeptide are encoded by the same gene, but may differ in their isoelectric point (=PI) or molecular weight (=MW), or both e.g., as a result of alternative mRNA or pre-mRNA processing. The amino acid sequence of a variant is to 95% or more identical to the corresponding marker sequence. In addition, or in the alternative a marker polypeptide or a variant thereof may carry a posttranslational modification. Preferred posttranslational modifications are glycosylation, acylation, and/or phosphorylation.

Preferably, the first or further markers as described herein are specifically measured from a sample by use of a specific binding agent.

A specific binding agent has at least an affinity of 10⁷l/mol for its corresponding target molecule. The specific binding agent preferably has an affinity of 10⁸ l/mol or also preferred of 10⁹ l/mol for its target molecule. As the skilled artisan will appreciate, the term specific is used to indicate that other biomolecules present in the sample do not significantly bind to the binding agent specific for the marker. Preferably, the level of binding to a biomolecule other than the target molecule results in a binding affinity which is at most only 10% or less, only 5% or less, only 2% or less or only 1% or less of the affinity to the target molecule, respectively. A preferred specific binding agent will fulfil both the above minimum criteria for affinity as well as for specificity.

A specific binding agent preferably is an antibody reactive with the first or further marker as described herein. The term antibody refers to a polyclonal antibody, a monoclonal antibody, antigen binding fragments of such antibodies, single chain antibodies as well as to genetic constructs comprising the binding domain of an antibody.

Any antibody fragment retaining the above criteria of a specific binding agent can be used. Antibodies are generated by state of the art procedures, e.g., as described in Tijssen (Tijssen, P., Practice and theory of enzyme immunoassays, 11, Elsevier Science Publishers B.V., Amsterdam, the whole book, especially pages 43-78). In addition, the skilled artisan is well aware of methods based on immunosorbents that can be used for the specific isolation of antibodies. By these means the quality of polyclonal antibodies and hence their performance in immunoassays can be enhanced. (Tijssen, P., *supra,* pages 108-115).

For the achievements as disclosed in the present invention polyclonal antibodies raised in rabbits may be used. However, clearly also polyclonal antibodies from different species, e.g., rats or guinea pigs, as well as monoclonal antibodies can also be used. Since monoclonal antibodies can be produced in any amount required with constant properties, they represent ideal tools in development of an assay for clinical routine. The generation and the use of monoclonal antibodies to the first or further marker as described herein in a method according to the present invention, respectively, represent yet other preferred embodiments.

As the skilled artisan will appreciate, now that the first or further marker as described herein has been identified as a marker which is useful in the assessment of an increased risk for mortality, various immunodiagnostic procedures may be used to reach a result comparable to the achievements of the present invention. For example, alternative strategies to generate antibodies may be used. Such strategies comprise amongst others the use of synthetic peptides, representing an epitope of the first or further marker as described herein for immunization. Alternatively, DNA immunization also known as DNA vaccination may be used.

For determining in the sample obtained from a subject said first or further marker, the sample is incubated with the specific binding agent for said first or further marker under conditions appropriate for formation of a binding agent marker-complex. Such conditions need not be specified, since the skilled artisan without any inventive effort can easily identify such appropriate incubation conditions. The amount of binding agent marker-complex is measured and used in the assessment of an increased risk for mortality. As the skilled artisan will appreciate there are numerous methods to measure the amount of the specific binding agent marker-complex all described in detail in relevant textbooks (cf., e.g., Tijssen P., *supra,* or Diamandis, E. P. and Christopoulos, T. K. (eds.), Immunoassay, Academic Press, Boston (1996)).

For example, the marker as described herein is detected in a sandwich type assay format. In such assay a first specific binding agent is used to capture the marker on the one side and a second specific binding agent, which is labeled to be directly or indirectly detectable, is used on the other side.

In a preferred embodiment, measurement of the marker as described herein in a sample is carried out by using a sandwich immunoassay, wherein Streptavidin-coated microtiter plates are used. A biotinylated polyclonal antibody to marker as described herein is used as a capturing antibody and a digoxigenylated polyclonal antibody to said marker is used as the second specific binding partner in this sandwich assay. The sandwich complex formed is finally visualized by an anti-digoxigenin horseradish peroxidase conjugate and an appropriate peroxidase substrate.

As mentioned above, the first or further marker can be determined from a liquid sample obtained from a subject sample.

In a preferred embodiment the method according to the present invention is practised with blood serum as liquid sample material.

The term "sample" as used herein refers to a biological sample obtained for the purpose of evaluation. In the methods of the present invention, the sample or subject's sample preferably may comprise any body fluid or a tissue extract. For example, test samples include blood, serum, plasma, cerebrospinal fluid and salvia. Preferred samples are whole blood, serum, or plasma, with serum being most preferred.

In one embodiment, the assessment is made in vitro. The subject sample is discarded afterwards. The subject sample is solely used for the in vitro method of the invention and the material of the subject sample is not transferred back into the subject's body. Typically, the sample is a liquid sample, e.g., whole blood, serum, or plasma, preferably serum.

Biochemical markers can either be determined individually or in a preferred embodiment of the invention they can be measured simultaneously using a chip or a bead based array technology. The concentrations of the biomarkers are then either interpreted independently, e.g., using an individual cut-off for each marker or reference amount, or they are combined for interpretation.

The data established in the present invention indicate that the present invention indicate that the markers alpha-Glutathione-S-Transferase, Trefoil Factor 3, alpha-2-Macroglobulin, Macrophage-derived Chemokine, Apolipoprotein B, Selenoprotein P, Tenascin C and Hepatocyte Growth Factor Receptor will form an integral part of a marker panel appropriate for diagnostic purposes optionally the amount of at least a further marker and wherein said further marker is selected from the group consisting of Nt-proBNP, Angiopoietin 2, Growth Differentiation Factor 15, Peroxiredoxin 4, YKL40, Insulin-like Growth Factor Binding Protein 2, Osteoprotegerin and Chromogranin A.

The data established in the present invention also indicate that the markers alpha-Glutathione-S-Transferase, Trefoil Factor 3, alpha-2-Macroglobulin, and Macrophage-derived Chemokine will form an integral part of a marker panel appropriate for diagnostic purposes.

The present invention therefore relates to the use of at least one first marker selected from the group consisting of alpha-Glutathione-S-Transferase, Trefoil Factor 3, alpha-2-Macroglobulin, Macrophage-derived Chemokine, Apolipoprotein B, Selenoprotein P, Tenascin C and Hepatocyte Growth Factor Receptor, and optionally at least one further marker as described herein in the assessment of an increased risk for mortality, preferably cardiovascular mortality, in a subject, wherein determining an altered amount of said first marker in a sample from the subject compared to a reference amount for said marker is indicative for said increased risk.

The invention also relates to the use of a marker panel comprising alpha-Glutathione-S-Transferase, Trefoil Factor 3, alpha-2-Macroglobulin, Macrophage-derived Chemokine, Apolipoprotein B, Selenoprotein P, Tenascin C and Hepatocyte Growth Factor Receptor, and optionally at least one further marker as described herein in the assessment of an increased risk for mortality, preferably a cardiovascular mortality in a subject, wherein determining an altered amount of at least alpha-Glutathione-S-Transferase, Trefoil Factor 3, alpha-2-Macroglobulin, Macrophage-derived Chemokine, Apolipoprotein B, Selenoprotein P, Tenascin C and Hepatocyte Growth Factor Receptor is indicative for said risk.

A preferred further marker as described herein is selected from the group consisting of Nt-proBNP, Angiopoietin-2, Growth Differentiation Factor 15, Peroxiredoxin-4, YKL-40, Insulin-like Growth Factor Binding Protein 2, Osteoprotegerin and Chromogranin A.

In a preferred embodiment the further marker as described herein is selected from the group consisting of Growth Differentiation Factor 15, Insulin-like Growth Factor Binding Protein 2, Angiopoietin 2, Nt-proBNP, YKL40, Osteoprotegerin, and Chromogranin A.

In one embodiment, the present invention relates to the use of at least one first marker selected from the group consisting of alpha-Glutathione-S-Transferase, Trefoil Factor 3, alpha-2-Macroglobulin, and Macrophage-derived Chemokine, and optionally at least one further marker as described herein in the assessment of an increased risk for mortality, preferably cardiovascular mortality, in a subject, wherein determining an altered amount of said first marker in a sample from the subject compared to a reference amount for said marker is indicative for said increased risk.

The invention also relates to the use of a marker panel comprising alpha-Glutathione-S-Transferase, Trefoil Factor 3, alpha-2-Macroglobulin, and Macrophage-derived Chemokine, and optionally at least one further marker as described herein in the assessment of an increased risk for mortality, preferably cardiovascular mortality, in a subject, wherein determining an altered amount of at least alpha-Glutathione-S-Transferase, Trefoil Factor 3, alpha-2-Macroglobulin, and/or Macrophage-derived Chemokine is indicative for said increased risk.

A preferred further marker in the use or the marker panel as described herein is selected from the group consisting of Nt-pro BNP, Peroxiredoxin-4, Growth Differentiation Factor-15, Angiopoietin-2 and YKL-40 and Insulin-like Growth Factor Binding Protein 2.

In a further embodiment the present invention relates to the use of at least one first marker selected from the group consisting of alpha-Glutathione-S-Transferase, Trefoil Factor 3, Macrophage-derived Chemokine, alpha-2-Macroglobulin, Selenoprotein P, and Tenascin C, and optionally at least one further marker as described herein in the assessment of an increased risk for mortality, preferably cardiovascular mortality in a subject, wherein determining an altered amount of said first marker in a sample from the subject compared to a reference amount for said marker is indicative for said risk.

In a further embodiment the invention also relates to the use of a marker panel comprising alpha-Glutathione-S-Transferase, Trefoil Factor 3, Macrophage-derived Chemokine, alpha-2-Macroglobulin, Selenoprotein P and Tenascin C, and optionally at least one further marker as described herein in the assessment of an increased risk for mortality, preferably cardiovascular mortality in a subject, wherein determining an altered amount of at least alpha-Glutathione-S-Transferase, Trefoil Factor 3, Macrophage-derived Chemokine, alpha-2-Macroglobulin, Selenoprotein P and/or Tenascin C is indicative for said increased risk.

In a preferred embodiment the further marker as described herein is selected from Growth Differentiation Factor 15, Insulin-like Growth Factor Binding Protein 2, Angiopoietin-2, Nt-pro BNP, YKL-40 and Chromogranin A.

In one embodiment, the invention relates to the use of at least one first marker as described herein wherein said marker is alpha-Glutathione-S-Transferase, optionally with at least a further marker, e.g., wherein said at least further marker is selected from the group consisting of Nt-pro BNP, Angiopoietin-2, Growth Differentiation Factor 15, Peroxiredoxin-4, YKL-40 and Insulin-like Growth Factor Binding Protein 2.

In a particularly preferred embodiment, the invention relates to the use of the first and further marker alpha-Glutathione-S-Transferase, Nt-pro BNP and Aniopoietin-2.

In a particularly preferred embodiment, the invention relates to the use of the first and further marker Hepatocyte Growth Factor Receptor and Chromogranin A.
In one embodiment, the invention relates to the use of at least one first marker as described herein wherein said marker is Trefoil Factor 3, optionally with at least a further marker, e.g., wherein said at least further marker is selected from the group consisting of Nt-pro BNP, Angiopoietin-2, Growth Differentiation Factor 15, Peroxiredoxin-4, YKL-40 and Insulin-like Growth Factor Binding Protein 2.

In one embodiment, the invention relates to the use of at least one first marker as described herein wherein said marker is alpha-2-Macroglobulin, optionally with at least a further marker, e.g., wherein said at least further marker is selected from the group consisting of Nt-pro BNP, Angiopoietin-2, Growth Differentiation Factor 15,Peroxiredoxin-4, YKL-40 and Insulin-like Growth Factor Binding Protein 2.

In one embodiment, the invention relates to the use of at least one first marker as described herein wherein said marker is Macrophage-derived Chemokine, optionally with at least a further marker, e.g., wherein said at least further marker is selected from the group consisting of Nt-pro BNP, Angiopoietin-2, Growth Differentiation Factor 15, Peroxiredoxin-4, YKL-40 and Insulin-like Growth Factor Binding Protein 2.

In one embodiment, the invention relates to the use of at least one first marker as described herein wherein said marker is selected from the group consisting of alpha-Glutathione-S-Transferase, alpha-2 Macroglobulin and Macrophage-derived Chemokine, optionally with at least a further marker, e.g., wherein said at least further marker is selected from the group consisting of Nt-pro BNP, Angiopoietin-2, Growth Differentiation Factor 15,Peroxiredoxin-4, YKL-40 and Insulin-like Growth Factor Binding Protein 2.

In one embodiment, the invention relates to the use of at least one first marker as described herein, wherein said first marker is selected from the group consisting of alpha-Glutathione-S-Transferase, alpha-2-Macroglobulin, Macrophage-derived Chemokine, Apolipoprotein B, Selenoprotein P, Tenascin C, and Hepatocyte Growth Factor Receptor, optionally with at least a further marker as described herein.

In one embodiment, the invention relates to the use of at least one first marker as described herein, wherein said first marker is selected from the group consisting of alpha-Glutathione-S-Transferase, alpha-2-Macroglobulin, Macrophage-derived Chemokine, Selenoprotein P and Tenascin C, optionally with at least a further marker as described herein.

In one embodiment, the invention relates to the use of at least one first marker as described herein wherein said marker is alpha-Glutathione-S-Transferase and alpha-2 Macroglobulin optionally with at least a further marker, e.g., wherein said at least further marker is selected from the group consisting of Nt-pro BNP, Angiopoietin-2, Growth Differentiation Factor 15, Peroxiredoxin-4, YKL-40 and Insulin-like Growth Factor Binding Protein 2.

In one embodiment, the invention relates to the use of at least one first marker as described herein wherein said marker is alpha-Glutathione-S-Transferase and Macrophage-derived Chemokine, optionally with at least a further marker, e.g., wherein said at least further marker is selected from the group consisting of Nt-pro BNP, Angiopoietin-2, Growth Differentiation Factor 15, Peroxiredoxin-4, YKL-40 and Insulin-like Growth Factor Binding Protein 2.

In one embodiment, the invention relates to the use of at least one first marker as described herein wherein said marker is alpha-2-Macroglobulin and Macrophage-derived Chemokine, optionally with at least a further marker, e.g., wherein said at least further marker is selected from the group consisting of Nt-pro BNP, Angiopoietin-2, Growth Differentiation Factor 15, Peroxiredoxin-4, YKL-40 and Insulin-like Growth Factor Binding Protein 2.

The use of a preferred marker panel of the invention as described herein comprises alpha-Glutathione-S-Transferase, Trefoil Factor 3, alpha-2-Macroglobulin, Macrophage-derived Chemokine, YKL-40, Insulin-like Growth Factor Binding Protein 2, Nt-pro BNP, Peroxiredoxin-4, Growth Differentiation Factor-15 and Angiopoietin-2.

The use of a particularly preferred marker panel of the invention as described herein comprises alpha-Glutathione-S-Transferase, Nt-pro BNP and Angiopoietin-2.
The use of a particularly preferred marker panel of the invention as described herein comprises Hepatocyte Growth Factor Receptor and a further marker Chromogranin A.

In one embodiment the invention relates to the use of at least one first marker, wherein said first marker is Apolipoprotein B, optionally with at least a further marker as described herein.

In one embodiment the invention relates to the use of at least one first marker, wherein said first marker is Selenoprotein P, optionally with at least a further marker as described herein.

In one embodiment the invention relates to the use of at least one first marker, wherein said first marker is Tenascin C, optionally with at least a further marker as described herein.

In one embodiment the invention relates to the use of at least one first marker, wherein said first marker is Hepatocyte Growth Factor Receptor, optionally with at least a further marker as described herein.

Another use of a preferred marker panel of the invention as described herein comprises alpha-Glutathione-S-Transferase, Trefoil Factor 3, alpha-2-Macroglobulin, Macrophage-derived Chemokine, Chromogranin A, Selenoprotein P, Tenascin C, Hepatocyte Growth Factor Receptor, Osteoprotegerin, Apolipoprotein B, Growth-Differentiation-Factor-15, Insulin-like Growth Factor Binding Protein 2, Angiopoietin-2, Nt-pro BNP, and YKL-40.

Another use of the preferred marker panel of the invention as described herein comprises alpha-Glutathione-S-Transferase, Trefoil Factor 3, Macrophage-derived Chemokine, alpha-2-Macroglobulin, Chromogranin A, Selenoprotein P, Tenascin C, Growth Differentiation Factor 15, Insulin-like Growth Factor Binding Protein 2, Angiopoietin-2, Nt-pro BNP, and YKL-40.
Another use of the preferred marker panel of the invention as described herein comprises alpha-Glutathione-S-Transferase, Trefoil Factor 3, Macrophage-derived Chemokine, alpha-2-Macroglobulin, Chromogranin A, Selenoprotein P, Tenascin C, Growth Differentiation Factor 15, Insulin-like Growth Factor Binding Protein 2, Angiopoietin-2, Nt-pro BNP, YKL-40, Apolipoprotein B, Hepatocyte Growth Factor Receptor, Peroxiredoxin 4, and Osteoprotegerin.

As the skilled artisan will appreciate, there are many ways to use the determination of the amount of two or more markers in order to correlate the diagnostic question under investigation. In a quite simple, but nonetheless often effective approach, a positive result, i.e. presence of an increased risk for mortality, is assumed if in a sample the marker is altered compared to a reference amount. For example, for the markers Trefoil Factor 3, alpha-2-Macroglobulin, Growth Differentiation Factor 15, Nt-pro BNP, Peroxiredoxin-4, YKL-40, Insulin-like Growth Factor Binding Protein 2 and Angiopoietin-2 a level above the reference amount predicts an increased risk for mortality. In contrast, for example, for the markers Glutathione-S-Transferase and Macrophage-derived Chemokine, a level above the reference amount predicts a decreased risk for mortality.

Frequently, however, the combination of markers is evaluated. Preferably the values measured for markers of a marker panel, e.g. for Trefoil Factor 3 and Nt-pro BNP, are mathematically combined and the combined value is correlated to the underlying diagnostic question. Marker values may be combined by any appropriate state of the art mathematical method. Well-known mathematical methods for correlating a marker combination to a disease employ methods like, discriminant analysis (DA) (i.e. linear-, quadratic-, regularized-DA), Kernel Methods (i.e. SVM), Nonparametric Methods (i.e. k-Nearest-Neighbor Classifiers), PLS (Partial Least Squares), Tree-Based Methods (i.e. Logic Regression, CART, Random Forest Methods, Boosting/Bagging Methods), Generalized Linear Models (i.e. Logistic Regression), Principal Components based Methods (i.e. SIMCA), Generalized Additive Models, Fuzzy Logic based Methods, Neural Networks and Genetic Algorithms based Methods. The skilled artisan will have no problem in selecting an appropriate method to evaluate a marker combination of the present invention. Details relating to these statistical methods are found in the following references: Ruczinski, I., et al, J. of Computational and Graphical Statistics, 12 (2003) 475-511; Friedman, J. H., J. of the American Statistical Association 84 (1989) 165-175; Hastie, Trevor, Tibshirani, Robert, Friedman, Jerome, The Elements of Statistical Learning, Springer Series in Statistics, 2001; Breiman, L., Friedman, J. H., Olshen, R. A., Stone, C. J. (1984) Classification and regression trees, California: Wadsworth; Breiman, L., Random Forests, Machine Learning, 45 (2001) 5-32; Pepe, M. S., The Statistical Evaluation of Medical Tests for Classification and Prediction, Oxford Statistical Science Series, 28 (2003); and Duda, R. O., Hart, P. E., Stork, D. G., Pattern Classification, Wiley Interscience, 2nd Edition (2001).

It is a further preferred embodiment of the invention to use an optimized multivariate cut-off for the underlying combination of biological markers and to discriminate state A from state B, e.g. risk from non-risk. In this type of analysis the markers are no longer independent but form a marker panel.

Accuracy of a diagnostic method is best described by its receiver-operating characteristics (ROC) (see especially Zweig, M. H., and Campbell, G., Clin. Chem. 39 (1993) 561-577). The ROC graph is a plot of all of the sensitivity/specificity pairs resulting from continuously varying the decision threshold over the entire range of data observed.

The clinical performance of a laboratory test depends on its diagnostic accuracy, or the ability to correctly classify subjects into clinically relevant subgroups. Diagnostic accuracy measures the test's ability to correctly distinguish two different conditions of the subjects investigated. Such conditions are for example health and disease or benign versus malignant disease.

In each case, the ROC plot depicts the overlap between the two distributions by plotting the sensitivity versus 1 - specificity for the complete range of decision thresholds. On the y-axis is sensitivity, or the true-positive fraction [defined as (number of true-positive test results)/(number of true-positive + number of false-negative test results)]. This has also been referred to as positivity in the presence of a disease or condition. It is calculated solely from the affected subgroup. On the x-axis is the false-positive fraction, or 1 - specificity [defined as (number of false-positive results)/(number of true-negative + number of false-positive results)]. It is an index of specificity and is calculated entirely from the unaffected subgroup. Because the true- and false-positive fractions are calculated entirely separately, by using the test results from two different subgroups, the ROC plot is independent of the prevalence of disease in the sample. Each point on the ROC plot represents a sensitivity/1-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test. One preferred way to quantify the diagnostic accuracy of a laboratory test is to express its performance by a single number. Such an overall parameter, e.g., is the so-called "total error" or alternatively the "area under the curve = AUC". The most common global measure is the area under the ROC plot. By convention, this area is always ≥ 0.5 (if it is not, one can reverse the decision rule to make it so). Values range between 1.0 (perfect separation of the test values of the two groups) and 0.5 (no apparent distributional difference between the two groups of test values). The area does not depend only on a particular portion of the plot such as the point closest to the diagonal or the sensitivity at 90% specificity, but on the entire plot. This is a quantitative, descriptive expression of how close the ROC plot is to the perfect one (area = 1.0).

Disclosed are systems and methods for developing diagnostic tests like, for example, a kit (e.g., detection, screening, monitoring, predictive and prognostic tests).

The invention also relates to a kit for performing the method of the invention comprising a reagent required to specifically determine at least alpha-Glutathione-S-Transferase, Trefoil Factor 3, alpha-2-Macroglobulin, Macrophage-derived Chemokine, Apolipoprotein B, Selenoprotein P, Tenascin C and Hepatocyte Growth Factor Receptor, and optionally at least a further marker and auxiliary reagents for performing the determination.

A preferred further marker as described herein is selected from the group consisting of Nt-pro BNP, Angiopoietin-2, Growth Differentiation Factor-15, Peroxiredoxin-4, YKL-40, Insulin-like Growth Factor Binding Protein 2, Osteoprotegerin and Chromogranin A.

A further preferred marker is preferably selected from the group consisting of Growth Differentiation Factor 15, Insulin-like Growth Factor Binding Protein 2, Angiopoietin 2, Nt-proBNP, YKL40, Osteoprotegerin, and Chromogranin A.

The invention also relates to a kit for performing the method of the invention comprising a reagent required to specifically determine at least alpha-Glutathione-S-Transferase, Trefoil Factor 3, alpha-2-Macroglobulin, and/or Macrophage-derived Chemokine, and optionally at least a further marker and auxiliary reagents for performing the determination.

The at least further marker as described herein is selected from the group consisting of Nt-proBNP, Peroxiredoxin-4, Growth Differentiation Factor-15, Angiopoietin-2, YKL-40 and Insulin-like Growth Factor Binding Protein 2.

In a further embodiment the invention also relates to a kit for performing the method of the invention comprising a reagent required to specifically determine at least alpha-Glutathione-S-Transferase, Trefoil Factor 3, Macrophage-derived Chemokine, alpha-2-Macroglobulin, Selenoprotein P, and Tenascin C, and optionally at least a further marker and auxiliary reagents for performing the determination.

A preferred further marker is selected from the group consisting of Growth Differentiation Factor-15, Insulin-like Growth Factor Binding Protein 2, Angiopoietin 2, Nt-proBNP, YKL40 and Chromogranin A.

The invention also relates to a kit for performing the method of the invention comprising a reagent required to specifically determine at least alpha-Glutathione-S-Transferase and optionally at least a further marker, e.g. selected from Nt-pro BNP, Peroxiredoxin-4, Growth Differentiation Factor-15 Angiopoietin-2, YKL-40 and Insulin-like Growth Factor Binding Protein 2, and optionally auxiliary reagents for performing the determination.

The invention also relates to a particularly preferred kit for performing the method of the invention comprising a reagent required to specifically determine at least alpha-Glutathione-S-Transferase, Nt-pro BNP and Angiopoietin-2, and optionally auxiliary reagents for performing the determination.

The invention also relates to a particularly preferred kit for performing the method of the invention comprising a reagent required to specifically determine at least Hepatocyte Growth Factor Receptor and Chromogranin A, and optionally auxiliary reagents for performing the determination.

The invention also relates to a kit for performing the method according to the invention comprising a reagent required to specifically determine at least Trefoil Factor 3 and optionally at least a further marker, e.g. selected from the group consisting of Nt-pro BNP, Peroxiredoxin-4, Growth Differentiation Factor-15 Angiopoietin-2, YKL-40 and Insulin-like Growth Factor Binding Protein 2, and optionally auxiliary reagents for performing the determination.

The invention also relates to a kit for performing the method of the invention comprising a reagent required to specifically determine at least alpha-2-Macroglobulin and optionally at least a further marker, e.g. selected from the group consisting of Nt-pro BNP, Peroxiredoxin-4, Growth Differentiation Factor-15,Angiopoietin-2, YKL-40 and Insulin-like Growth Factor Binding Protein 2, and optionally auxiliary reagents for performing the determination.

The invention also relates to a kit for performing the method of the invention comprising a reagent required to specifically determine at least Macrophage-derived Chemokine and optionally at least the further marker, e.g. selected from the group consisting of Nt-pro BNP, Peroxiredoxin-4, Growth Differentiation Factor-15, Angiopoietin-2, YKL-40 and Insulin-like Growth Factor Binding Protein 2, and optionally auxiliary reagents for performing the determination.

The invention also relates to a kit for performing the method of the invention comprising a reagent required to specifically determine at least alpha-Glutathione-S-Transferase, alpha-2 Macroglobulin and/or Macrophage-derived Chemokine, and optionally at least a further marker, e.g. selected from the group consisting of Nt-pro BNP, Peroxiredoxin-4, Growth Differentiation Factor-15, Angiopoietin-2, YKL-40 and Insulin-like Growth Factor Binding Protein 2, and optionally auxiliary reagents for performing the determination.

The invention also relates to a kit for performing the method of the invention comprising a reagent required to specifically determine at least alpha-Glutathione-S-Transferase and alpha-2 Macroglobulin, and optionally at least a further marker, e.g. selected from the group consisting of Nt-pro BNP, Peroxiredoxin-4, Growth Differentiation Factor-15, Angiopoietin-2, YKL-40 and Insulin-like Growth Factor Binding Protein 2, and optionally auxiliary reagents for performing the determination.

The invention also relates to a kit for performing the method of the invention comprising a reagent required to specifically determine at least alpha-Glutathione-S-Transferase and Macrophage-derived Chemokine, and optionally at least a further marker, e.g. selected from the group consisting of Nt-pro BNP, Peroxiredoxin-4, Growth Differentiation Factor-15, Angiopoietin-2, YKL-40 and Insulin-like Growth Factor Binding Protein 2, and optionally auxiliary reagents for performing the determination.

The invention also relates to a kit for performing the method of the invention comprising a reagent required to specifically determine at least alpha-2-Macroglobulin and Macrophage-derived Chemokine, and optionally at least a further marker, e.g. selected from the group consisting of Nt-pro BNP, Peroxiredoxin-4, Growth Differentiation Factor-15, Angiopoietin-2, YKL-40 and Insulin-like Growth Factor Binding Protein 2, and optionally auxiliary reagents for performing the determination.

The invention also relates to a kit for performing the method of the invention comprising a reagent required to specifically determine at least alpha-Glutathione-S-Transferase, Trefoil Factor 3, alpha-2-Macroglobulin, Macrophage-derived Chemokine, Nt-pro BNP, Angiopoietin-2, Growth-Differentiation-Factor-15, Peroxiredoxin 4, YKL-40 and Insulin-like Growth Factor Binding Protein 2, optionally auxiliary reagents for performing the determination.

The invention also relates to a kit for performing the method according to the invention comprising a reagent required to specifically determine at least Apolipoprotein B, and optionally at least a further marker as described herein.

The invention also relates to a kit for performing the method according to the invention comprising a reagent required to specifically determine at least Selenoprotein P, and optionally at least a further marker as described herein.

The invention also relates to a kit for performing the method according to the invention comprising a reagent required to specifically determine at least Tenascin C, and optionally at least a further marker as described herein.

The invention also relates to a kit for performing the method according to the invention comprising a reagent required to specifically determine at least Hepatocyte Growth Factor Receptor, and optionally at least a further marker as described herein.

The invention also relates to a kit for performing the method of the invention comprising a reagent required to specifically determine at least alpha-Glutathione-S-Transferase, alpha-2-Macroglobulin, Macrophage-derived Chemokine, Apolipoprotein B, Selenoprotein P, Tenascin C and/or Hepatocyte Growth Factor Receptor, and optionally at least a further marker as described herein.

The invention also relates to a kit for performing the method of the invention comprising a reagent required to specifically determine at least alpha-Glutathione-S-Transferase, Trefoil Factor 3, alpha-2-Macroglobulin, Macrophage-derived Chemokine, Chromogranin A, Selenoprotein P, Tenascin C, Hepatocyte Growth Factor Receptor, Osteoprotegerin, Apolipoprotein B, Growth-Differentiation-Factor-15, Insulin-like Growth Factor Binding Protein 2, Angiopoietin-2, Nt-pro BNP, and YKL-40.

The invention also relates to a kit for performing the method of the invention comprising a reagent required to specifically determine at least alpha-Glutathione-S-Transferase, Trefoil Factor 3, alpha-2-Macroglobulin, Macrophage-derived Chemokine, Chromogranin A, Selenoprotein P, Tenascin C, Hepatocyte Growth Factor Receptor, Osteoprotegerin, Peroxiredoxin-4, Chromogranin A, Growth-Differentiation-Factor-15, Insulin-like Growth Factor Binding Protein 2, Angiopoietin-2, Nt-pro BNP, and YKL-40.

The invention also relates to a kit for performing the method of the invention comprising a reagent required to specifically determine at least Tenascin C, alpha-Glutathione-S-Transferase, Macrophage-derived Chemokine, alpha-2-Macroglobulin and Selenoprotein P, and optionally at least a further marker as described herein.

The invention also relates to a kit for performing the method of the invention comprising a reagent required to specifically determine at least alpha-Glutathione-S-Transferase, Trefoil Factor 3, Macrophage-derived Chemokine, alpha-2-Macroglobulin, Chromogranin A, Selenoprotein P, Tenascin C, Growth-Differentiation-Factor-15, Insulin-like Growth Factor Binding Protein 2, Angiopoietin-2, Nt-pro BNP, and YKL-40.

The inventors also observed in the patient pool of the ORIGIN study receiving the insulin analogue insulin glargine and having a decreased level of angiopoietin-2, a reduced mortality as described herein.

An "insulin analogue" as used throughout the application refers to a polypeptide which has a molecular structure which formally can be derived from the structure of a naturally occurring insulin, for example that of human insulin, by deleting and/or exchanging at least one amino acid residue occurring in the naturally occurring insulin and/or adding at least one amino acid residue. The added and/or exchanged amino acid residue can either be codable amino acid residues or other naturally occurring residues or purely synthetic amino acid residues. Examples of analogues of insulin include, but are not limited to, the following:
(i). 'Insulin aspart' is created through recombinant DNA technology so that the amino acid B28 in human insulin (i.e. the amino acid no. 28 in the B chain of human insulin), which is proline, is replaced by
   aspartic acid;
(ii). 'Insulin lispro' is created through recombinant DNA technology so that the penultimate lysine and proline residues on the C-terminal end of the B-chain of human insulin are reversed (human insulin:
   ProB28LysB29; insulin lispro: LysB28ProB29);
(iii). 'Insulin glulisine' differs from human insulin in that the amino acid asparagine at position B3 is replaced by lysine and the lysine in position B29 is replaced by glutamic acid;
(iv). "Insulin glargine" differs from human insulin in that the asparagine at position A21 is replaced by glycine and the B chain is extended at the carboxy terminal by two arginines.

A preferred insulin analogue of the invention is insulin glargine.

In view of that, a further aspect of the invention pertains to an insulin analogue, e.g., insulin glargine for use in reducing mortality, particularly cardiovascular mortality as described herein, wherein said subject expresses a reduced amount of angiopoietin-2 compared to a reference amount as described herein. Preferably, the subject is pre-diabetic or diabetic as described herein. In an alternative preferred embodiment, the subject is pre-diabetic or diabetic as described herein and has an age of at least 50 years, preferably 55 years, 60 years, 63 years, or 65 years.

In an alternative preferred embodiment, the subject is pre-diabetic or diabetic as described herein and had a previous cardiovascular disorder as described herein.

More preferably, the subject is pre-diabetic or diabetic as described herein, has an age of at least 50 years, preferably 55 years, 60 years, 63 years, or 65 years, and had a previous cardiovascular disorder as described herein.

### Examples

### Example I

### 1. Creation of a Model Building and Validation Set

As noted in the approved SAP, the 8401 individuals were divided into 2 groups stratified by region: a) model building (67%) and b) validation (33%) group. As no bloods were available from China, The regions were agreed upon as follows: North America and Australia; South America: Europe (including South Africa); and India. The characteristics of the participants in each set are noted below.

| **Table 1: All ORIGIN Participants** | | | | | | | |
|---|---|---|---|---|---|---|---|
| | | Overall | | Glargine | | Standard Care | |
| | N | N/Mean | %/SD | N/Mean | %/SD | N/Mean | %/SD |
| Categorical Variables | | | | | | | |
| --N. America + Australia | 12537 | 1516 | 12.1 | 762 | 12.2 | 754 | 12.0 |
| --S. America | 12537 | 3853 | 30.7 | 1925 | 30.7 | 1928 | 30.7 |
| --Europe | 12537 | 6060 | 48.3 | 3027 | 48.3 | 3033 | 48.4 |
| --India | 12537 | 390 | 3.1 | 194 | 3.1 | 196 | 3.1 |
| Prior CV Event* | 12533 | 7378 | 58.9 | 3712 | 59.3 | 3666 | 58.4 |
| Reported or measured Microalb/Alb* | 12537 | 3968 | 31.7 | 1984 | 31.7 | 1984 | 31.6 |
| Male | 12536 | 8150 | 65.0 | 4181 | 66.8 | 3969 | 63.3 |
| Male>=55y or female >=65y* | 12537 | 8765 | 69.9 | 4432 | 70.8 | 4333 | 69.1 |
| Current Smoking* | 12533 | 1552 | 12.4 | 781 | 12.5 | 771 | 12.3 |
| Prior diabetes* | 12536 | 10321 | 82.3 | 5162 | 82.4 | 5159 | 82.2 |
| Hypertension* | 12533 | 9963 | 79.5 | 4974 | 79.5 | 4989 | 79.5 |
| Age | 12537 | 63.05 | 7.82 | 63.05 | 7.79 | 63.04 | 7.85 |

| Continuous Variables | | | | | | | |
|---|---|---|---|---|---|---|---|
| --Cholesterol (mmol/L) | 12521 | 4.90 | 1.20 | 4.91 | 1.20 | 4.90 | 1.20 |
| --LDL Cholesterol (mmol/L)* | 12328 | 2.90 | 1.03 | 2.91 | 1.04 | 2.90 | 1.03 |
| --HDL Cholesterol (mmol/L) | 12471 | 1.19 | 0.32 | 1.19 | 0.32 | 1.20 | 0.32 |

| Outcome Variables | | | | | | | |
|---|---|---|---|---|---|---|---|
| Coprimary outcome 1 | 12537 | 2054 | 16.4 | 1041 | 16.6 | 1013 | 16.1 |
| Coprimary outcome 2 | 12537 | 3519 | 28.1 | 1792 | 28.6 | 1727 | 27.5 |
| Microvascular | 12537 | 2686 | 21.4 | 1323 | 21.1 | 1363 | 21.7 |
| New Diabetes | 12536 | 760 | 6.1 | 365 | 5.8 | 395 | 6.3 |
| Death | 12537 | 1916 | 15.3 | 951 | 15.2 | 965 | 15.4 |
| A1C <6% at 2 year visit | 12537 | 5729 | 45.7 | 3362 | 53.7 | 2367 | 37.7 |

| **Table 2: ORIGIN Biomarker Participants** | | | | | | | |
|---|---|---|---|---|---|---|---|
| | | Overall | | Glargine | | Standard Care | |
| | N | N/Mean | %/SD | N/Mean | %/SD | N/Mean | %/SD |
| Categorical Variables | | | | | | | |
| --N. America + Australia | 8401 | 1425 | 17.0 | 710 | 16.9 | 715 | 17.0 |
| --S. America | 8401 | 2772 | 33.0 | 1388 | 33.1 | 1384 | 32.9 |
| --Europe | 8401 | 3822 | 45.5 | 1903 | 45.4 | 1919 | 45.6 |
| --India | 8401 | 382 | 4.5 | 191 | 4.6 | 191 | 4.5 |
| Prior CV Event* | 8400 | 4991 | 59.4 | 2513 | 60.0 | 2478 | 58.9 |
| Reported or measured Microalb/Alb* | 8401 | 2656 | 31.6 | 1330 | 31.7 | 1326 | 31.5 |
| Male | 8401 | 5553 | 66.1 | 2834 | 67.6 | 2719 | 64.6 |
| Male>=55y or female >=65y* | 8401 | 5928 | 70.6 | 2997 | 71.5 | 2931 | 69.6 |
| Current Smoking* | 8400 | 1050 | 12.5 | 525 | 12.5 | 525 | 12.5 |
| Prior diabetes* | 8401 | 6840 | 81.4 | 3422 | 81.6 | 3418 | 81.2 |
| Hypertension* | 8400 | 6638 | 79.0 | 3320 | 79.2 | 3318 | 78.8 |
| Age | 8401 | 63.21 | 7.94 | 63.22 | 7.93 | 63.20 | 7.95 |

| Continuous Variables | | | | | | | |
|---|---|---|---|---|---|---|---|
| --Cholesterol (mmol/L) | 8393 | 4.89 | 1.18 | 4.89 | 1.17 | 4.89 | 1.18 |
| --LDL Cholesterol (mmol/L)* | 8278 | 2.90 | 1.03 | 2.90 | 1.03 | 2.89 | 1.02 |
| --HDL Cholesterol (mmol/L) | 8370 | 1.18 | 0.32 | 1.17 | 0.31 | 1.18 | 0.32 |

| Outcome Variables | | | | | | | |
|---|---|---|---|---|---|---|---|
| Coprimary outcome 1 | 8401 | 1405 | 16.7 | 727 | 17.3 | 678 | 16.1 |
| Coprimary outcome 2 | 8401 | 2435 | 29.0 | 1245 | 29.7 | 1190 | 28.3 |
| Microvascular | 8401 | 1794 | 21.4 | 887 | 21.2 | 907 | 21.5 |
| New Diabetes | 8401 | 550 | 6.5 | 259 | 6.2 | 291 | 6.9 |
| Death | 8401 | 1340 | 16.0 | 672 | 16.0 | 668 | 15.9 |
| A1C <6% at 2 year visit | 8401 | 4042 | 48.1 | 2389 | 57.0 | 1653 | 39.3 |

| **Table 3: Biomarker Participants in Model Building Group** | | | | | | | |
|---|---|---|---|---|---|---|---|
| | | Overall | | Glargine | | Standard Care | |
| | N | N/Mean | %/SD | N/Mean | %/SD | N/Mean | %/SD |
| Categorical Variables | | | | | | | |
| --N. America + Australia | 5630 | 955 | 17.0 | 491 | 17.3 | 464 | 16.6 |
| --S. America | 5630 | 1858 | 33.0 | 954 | 33.7 | 904 | 32.3 |
| --Europe | 5630 | 2561 | 45.5 | 1263 | 44.6 | 1298 | 46.4 |
| --India | 5630 | 256 | 4.5 | 123 | 4.3 | 133 | 4.8 |
| Prior CV Event* | 5630 | 3327 | 59.1 | 1680 | 59.3 | 1647 | 58.8 |
| Reported or measured Microalb/Alb* | 5630 | 1792 | 31.8 | 897 | 31.7 | 895 | 32.0 |
| Male | 5630 | 3680 | 65.4 | 1897 | 67.0 | 1783 | 63.7 |
| Male>=55y or female >=65y* | 5630 | 4003 | 71.1 | 2030 | 71.7 | 1973 | 70.5 |
| Current Smoking* | 5630 | 693 | 12.3 | 354 | 12.5 | 339 | 12.1 |
| Prior diabetes* | 5630 | 4590 | 81.5 | 2316 | 81.8 | 2274 | 81.2 |
| Hypertension* | 5630 | 4445 | 79.0 | 2242 | 79.2 | 2203 | 78.7 |
| Age | 5630 | 63.32 | 7.89 | 63.29 | 7.92 | 63.35 | 7.86 |

| Continuous Variables | | | | | | | |
|---|---|---|---|---|---|---|---|
| --Cholesterol (mmol/L) | 5623 | 4.90 | 1.19 | 4.91 | 1.18 | 4.89 | 1.21 |
| --LDL Cholesterol (mmol/L)* | 5550 | 2.91 | 1.03 | 2.92 | 1.04 | 2.89 | 1.03 |
| --HDL Cholesterol (mmol/L) | 5605 | 1.18 | 0.32 | 1.18 | 0.32 | 1.18 | 0.31 |

| Outcome Variables | | | | | | | |
|---|---|---|---|---|---|---|---|
| Coprimary outcome 1 | 5630 | 932 | 16.6 | 496 | 17.5 | 436 | 15.6 |
| Coprimary outcome 2 | 5630 | 1609 | 28.6 | 851 | 30.1 | 758 | 27.1 |
| Microvascular | 5630 | 1201 | 21.3 | 609 | 21.5 | 592 | 21.2 |
| New Diabetes | 5630 | 363 | 6.4 | 172 | 6.1 | 191 | 6.8 |
| Death | 5630 | 892 | 15.8 | 451 | 15.9 | 441 | 15.8 |
| A1C <6% at 2 year visit | 5630 | 2710 | 48.1 | 1626 | 57.4 | 1084 | 38.7 |

| **Table 4: Biomarker Participants in Validation Group** | | | | | | | |
|---|---|---|---|---|---|---|---|
| | | Overall | | Glargine | | Standard Care | |
| | N | N/Mean | %/SD | N/Mean | %/SD | N/Mean | %/SD |
| Categorical Variables | | | | | | | |
| --N. America + Australia | 2771 | 470 | 17.0 | 219 | 16.1 | 251 | 17.8 |
| --S. America | 2771 | 914 | 33.0 | 434 | 31.9 | 480 | 34.0 |
| --Europe | 2771 | 1261 | 45.5 | 640 | 47.0 | 621 | 44.0 |
| --India | 2771 | 126 | 4.5 | 68 | 5.0 | 58 | 4.1 |
| Prior CV Event* | 2770 | 1664 | 60.1 | 833 | 61.3 | 831 | 58.9 |
| Reported or measured Microalb/Alb* | 2771 | 864 | 31.2 | 433 | 31.8 | 431 | 30.6 |
| Male | 2771 | 1873 | 67.6 | 937 | 68.8 | 936 | 66.4 |
| Male>=55y or female >=65y* | 2771 | 1925 | 69.5 | 967 | 71.1 | 958 | 67.9 |
| Current Smoking* | 2770 | 357 | 12.9 | 171 | 12.6 | 186 | 13.2 |
| Prior diabetes* | 2771 | 2250 | 81.2 | 1106 | 81.3 | 1144 | 81.1 |
| Hypertension* | 2770 | 2193 | 79.2 | 1078 | 79.3 | 1115 | 79.1 |
| Age | 2771 | 63.00 | 8.04 | 63.08 | 7.96 | 62.92 | 8.12 |

| Continuous Variables | | | | | | | |
|---|---|---|---|---|---|---|---|
| --Cholesterol (mmol/L) | 2770 | 4.87 | 1.14 | 4.85 | 1.15 | 4.90 | 1.14 |
| --LDL Cholesterol (mmol/L)* | 2728 | 2.87 | 1.02 | 2.85 | 1.03 | 2.90 | 1.01 |
| --HDL Cholesterol (mmol/L) | 2765 | 1.18 | 0.31 | 1.16 | 0.30 | 1.19 | 0.33 |

| Outcome Variables | | | | | | | |
|---|---|---|---|---|---|---|---|
| Coprimary outcome 1 | 2771 | 473 | 17.1 | 231 | 17.0 | 242 | 17.2 |
| Coprimary outcome 2 | 2771 | 826 | 29.8 | 394 | 28.9 | 432 | 30.6 |
| Microvascular | 2771 | 593 | 21.4 | 278 | 20.4 | 315 | 22.3 |
| New Diabetes | 2771 | 187 | 6.7 | 87 | 6.4 | 100 | 7.1 |
| Death | 2771 | 448 | 16.2 | 221 | 16.2 | 227 | 16.1 |
| A1C <6% at 2 year visit | 2771 | 1332 | 48.1 | 763 | 56.1 | 569 | 40.4 |

### 2. Independent Predictors of Mortality When Added to the Basic Clinical Model

1. Because the final biomarker list included 237 of the 284 biomarkers originally assayed, the p value for inclusion in the models in the SAP was increased from 0.05/284=0.00018 to 0.05/237 = 0.00021.
2. It was recognized that the sex and age criterion in the clinical model only adjusted for age (i.e. sex-specific age) by dichotomizing the age variable. Because sex was also considered important and may not have emerged well in the matched INTERHEART study, we agreed to adjust for sex in the model-building cohort. With respect to smoking, second-hand smoke was not included in the model as that data was not collected, and the smoking variable was simplified to current versus non-smoker.
3. The final variables forced into the basic clinical model before assessing any biomarkers were therefore the following (based on the SAP):

| | |
|---|---|
| a) Prior CV outcome (Y/N) | e) LDL cholesterol/HDL cholesterol |
| b) Reported/measured albuminuria (Y/N) | f) Current Smoker (Y/N) |
| c) Male ≥ 55 y or female ≥ 65 y (Y/N) | g) Prior Diabetes (Y/N) |
| d) Sex (M/F) | h) Prior Hypertension (Y/N) |

4. To assess the possibility that analyzing the ordinal and continuous data in the same model disadvantages the ordinal data which has 5 levels versus the continuous data which has infinite possible levels, a sensitivity analysis was run in which the raw, non-transformed data for every one of the 192 continuous variables was used to divide the data into 5ths using quintiles and the model was rerun. The results were compared to the model that mixed continuous and ordinal data.
a. When the ordinal and continuous biomarkers were included in the same analysis, these biomarkers were significant at P<0.05/237 when added to the clinical model:
   i. Growth/differentiation factor 15
   ii. Insulin-like Growth Factor-Binding Protein 2
   iii. Angiopoietin-2
   iv. Glutathione S-Transferase alpha
   v. YKL-40
   vi. N-t pro BNP (ordinal)
   vii. Alpha-2-Macroglobulin
   viii. Macrophage-Derived Chemokine
   ix. Trefoil Factor 3
   x. Peroxiredoxin-4
b. When the raw values of the continuous biomarkers were converted to 5 ordinal levels and added to the 45 ordinal biomarkers, the same biomarkers were significant at P<0.05/237 when added to the clinical model; the order of entry was slightly different however.
   i. Insulin-like Growth Factor-Binding Protein 2
   ii. Growth/differentiation factor 15
   iii. N-t pro BNP (ordinal)
   iv. Peroxiredoxin-4
   v. Gutathione S-Transferase alpha
   vi. VEGF D (ordinal)
   vii. YKL-40
   viii. Visfatin
   ix. Pancreatic Polypeptide
   x. B Lymphocyte Chemoattractant (ordinal)
   xi. Adiponectin

As the first model included both ordinal and continuous variables and made the fewest assumptions it was retained. However the global test for proportionality for that model was significant (p=0.02) suggesting that the 1^{st} model did not satisfy the proportionality assumption. And within that model the following variables were not proportional based on the supremum test at p<0.05:
a) Hypertension
b) Glutathione S-Transferase alpha
c) N-t pro BNP

2. This was explored by repeating the first model using logistic regression instead of a Cox model. The exact same biomarkers were identified using that approach in the same order:
   i. Growth/differentiation factor 15
   ii. Insulin-like Growth Factor-Binding Protein 2
   iii. Angiopoietin-2
   iv. Glutathione S-Transferase alpha
   v. YKL-40
   vi. N-t pro BNP (ordinal)
   vii. Alpha-2-Macroglobulin
   viii. Macrophage-Derived Chemokine
   ix. Trefoil Factor 3
   x. Peroxiredoxin-4
3. This provided reassurance regarding the included biomarkers. However, as it was important to account for the proportionality we then repeated the Cox model but also included 3 additional terms: an interaction term of time with a) hypertension; b) glutathione S-Transferase alpha; and c) N-t pro BNP
4. Using this approach the final model is noted below:

**Table 5**

| **Cox Regression Including time Interaction Variables to Correct for Non-proportionality Identifying Independent Biomarker Predictors of Mortality when 237 Biomarkers (192 Continuous & 45 Ordinal) were Added to the Basic Clinical Model using the Model Building Set** | | | | | | | | | |
|---|---|---|---|---|---|---|---|---|---|
| **Parameter** | **Label** | **Parameter Estimate** | **SE** | **SD** | **HR** | **Lower Bond** | **Upper Bond** | **P value** | **Inclusion p value** |
| pCV | Prior CV Event | 0.09822 | 0.07654 | . | 1.103 | 0.95 | 1.282 | 1.99E-01 | . |
| microalb | Albuminuria | -0.10681 | 0.07466 | . | 0.899 | 0.776 | 1.04 | 1.53E-01 | . |
| SVSEX | Male | 0.14137 | 0.08397 | . | 1.152 | 0.977 | 1.358 | 9.23E-02 | . |
| gen_age | M>=55, F>=65 | 0.43398 | 0.10673 | . | 1.543 | 1.252 | 1.903 | 4.78E-05 | . |
| LDL_HDL | LDL/HDL | 0.13664 | 0.0305 | 1.161 | 1.146 | 1.08 | 1.217 | 7.45E-06 | . |
| Smk | Current Smoking | 0.52067 | 0.09753 | . | 1.683 | 1.39 | 2.038 | 9.36E-08 | . |
| Pdiab | Prior diabetes | 0.20447 | 0.09921 | . | 1.227 | 1.01 | 1.49 | 3.93E-02 | . |
| Hypten | Hypertension | -1.78203 | 0.67003 | . | 1.43* | | | 7.82E-03 | . |
| var101 | GDF 15 | 0.20844 | 0.04395 | 0.42 | 1.232 | 1.13 | 1.343 | 2.11E-06 | 0.00E+00 |
| var121 | IGF BP2 | 0.11956 | 0.04316 | 53.987 | 1.127 | 1.036 | 1.226 | 5.61 E-03 | 0.00E+00 |
| var14 | Angiopoietin-2 | 0.18026 | 0.03594 | 3.681 | 1.198 | 1.116 | 1.285 | 5.28E-07 | 8.99E-15 |
| var95 | Glut S-trans alpha | -0.5974 | 0.2983 | 31.193 | 0.83* | 0.77* | 0.89* | 4.52E-02 | 1.11E-09 |
| var283 | YKL-40 | 0.15968 | 0.03472 | 76.692 | 1.173 | 1.096 | 1.256 | 4.25E-06 | 2.57E-09 |
| var190 | N-t pro BNP | 0.86485 | 0.24409 | Ordinal | 1.17* | 1.10* | 1.24* | 3.95E-04 | 1.61E-08 |
| var11 | Alpha2 macroglob | 0.20033 | 0.03619 | 2.678 | 1.222 | 1.138 | 1.312 | 3.12E-08 | 1.13E-06 |
| var169 | M-derived chemok | -0.17274 | 0.03529 | 197.558 | 0.841 | 0.785 | 0.902 | 9.85E-07 | 1.21E-05 |
| var262 | Trefoil Factor 3 | 0.20193 | 0.04147 | 0.108 | 1.224 | 1.128 | 1.327 | 1.12E-06 | 4.17E-06 |
| var207 | Peroxiredoxin-4 | 0.13183 | 0.0344 | 1.39 | 1.141 | 1.067 | 1.22 | 1.27E-04 | 1.36E-04 |
| Hypten*t | Time interaction | 0.28535 | 0.09704 | . | 1.33 | 1.1 | 1.609 | 3.28E-03 | . |
| var95*t | Time interaction | 0.05442 | 0.04242 | . | 1.056 | 0.972 | 1.147 | 2.00E-01 | . |
| var190*t | Time interaction | -0.0943 | 0.03444 | . | 0.91 | 0.851 | 0.974 | 6.18E-03 | . |

| | | | | | | | | | |
|---|---|---|---|---|---|---|---|---|---|
| P for inclusion < 0.05/237 or 0.00021097; Hazard ratios are expressed per 1 SD change in the parameter (NB - the SD is the SD of the transformed value if the variable was transformed because of non-normality); *this HR is based on 5 yrs f/u due to the time interaction | | | | | | | | | |

The adjustment for non-proportionality also means that the estimate of the effect of the biomarker is different at different time points and the time has to be taken into account. Therefore for the 3 interacting variables (hypertension, glutathione S-Transferase alpha, and Nt-pro BNP), the HR at 1, 3 and 5 years is noted below:

| **Hazard Ratios by Time Since Randomization for the 3 Time- Interacting Variables** | | | |
|---|---|---|---|
| **Time** | **Glut-S-Transferase** | | |
| | **Hypertension HR** | **Alpha HR** | **N-t pro BNP HR** |
| Year 1 | 0.91 | 0.76 | 1.36 |
| Year 3 | 1.24 | 0.81 | 1.23 |
| Year 5 | 1.43 | 0.83 | 1.17 |

The diagnostic properties of the Cox Model shown above are as follows:
**a) Likelihood Ratio Test:** Adding biomarkers to the basic clinical model increased the model chi-square from 245 to 864 (LR test =619 df=10 with p<0.001).
**b) C statistic with 95 %Cl will depend on the time after study entry in which the risk is assessed so is reported at 2 time points arbitrarily chosen:**
   Year 1: clinical model = 0.64 (0.63, 0.66); clinical model + biomarkers = 0.76 (0.74, 0.77)
   Year 4: clinical model = 0.65 (0.63, 0.66); clinical model + biomarkers = 0.76 (0.75, 0.78)
   Max F/U: clinical model = 0.64 (0.63, 0.66); clinical model + biomarkers = 0.76 (0.74, 0.78)
**c) Model calibration (Hosmer-Lemeshow) with 4 categories of risk probabilities (<5%, 5-10%, 10-20%, <20%):**
   Chi square from 108 to 6 at year 1(p=0.058)
   Chi square from 358 to 19 at year 4 (p=0.089)
   Chi Square from 438 to 56 at max survival time (7.84 yrs) (p=6.35E-9)
d) **Net reclassification Index (NRI)** using Bootstrap method
   1.37 (95%CI 0.23, 0.50) at year 1
   0.54 (95%CI 0.47, 0.61) at year 4
   0.38 (95%CI 0.34, 0.43) at max survival time (7.84 yrs)

### 3. Validation of Results

Using the validation set, a C statistic for both the clinical and clinical + biomarker models (identified in the model building sets), as well as the NRI were calculated for mortality. Sensitivity and specificity for the cut-point that maximized these 2 values was also calculated. Forward selection was not done in the validation set. The following table shows the results of these analyses.

| | **C stat (95% CI)** | **Cut-point** | **Sensitivity** | **Specificity** | **NRI(95% CI)** |
|---|---|---|---|---|---|
| ***Mortality*** | | | | | |
| Model Building: Clinical | 0.64(0.63- 0.66) | 40 | 0.78 | 0.45 | |
| Model Building: Full | 0.76(0.74- 0.78) | 60 | 0.74 | 0.67 | 0.37(0.34- 0.41) |
| Validation: Clinical | 0.63(0.61- 0.66) | 70 | 0.47 | 0.74 | |
| Validation: Full | 0.73(0.70- 0.75) | 70 | 0.59 | 0.76 | 0.32(0.26- 0.38) |

| **Model Performance of Participants in the ORIGIN Biomarker Study** | | | |
|---|---|---|---|
| | **Basic Clinical C (95%CI)** | **Full Model C (95% CI)** | **Improvement NRI (95%CI)** |
| ***Death from All Causes*** | | | |
| Model Building Set | 0.64 (0.63, 0.66) | 0.76 (0.74, 0.78) | 0.37 (0.34, 0.41) |
| Validation Set* | 0.63 (0.61, 0.66) | 0.73 (0.70, 0.75) | 0.32 (0.26, 0.38) |
| All Participants | 0.64 (0.63, 0.66) | 0.76 (0.75, 0.77) | 0.32 (0.29. 0.36) |

| | | | |
|---|---|---|---|
| *the model built with the model building set for the outcome was tested using the validation set | | | |

Conclusions from these analyses were that the validation set yielded findings consistent with the model building set.

4. For the final validation the forward selection process was repeated with the full 8401 participants for mortality. These models, as well as estimates of hazard ratios using sensitivity analyses in which age, creatinine, and both age and creatinine are added to the basic clinical model are shown below for each outcome.

Also shown below is an estimate of the HR and Cl for each of the biomarkers identified in the run of all 8401, as well as the C statistics and NRI for each model that was derived from running the clinical model 1000 times and the clinical + biomarker model 1000 times using bootstrapping (i.e. 1000 samples of 8401 randomly drawn with replacement).

| **Biomarkers for Mortality Detected with Forward Selection on 8401 Pts** | | | | | |
|---|---|---|---|---|---|
| **Parameter** | **Label** | **SD** | **HR** | **HR ICI** | **HR uCI** |
| pCV | Prior CV Event | . | 1.18 | 1.04 | 1.33 |
| microalb | Microalb/Alb | . | 0.97 | 0.86 | 1.10 |
| SVSEX | | . | 1.23 | 1.07 | 1.41 |
| gen_age | M>=55y/F >=65y | . | 1.37 | 1.16 | 1.62 |
| LDL_HDL | LDL/HDL | 1.161 | 1.09 | 1.03 | 1.16 |
| smk | Current Smoking | . | 1.55 | 1.33 | 1.81 |
| pdiab | Prior diabetes | . | 1.24 | 1.06 | 1.45 |
| hypten | Hypertension | . | 1.10 | 0.95 | 1.28 |
| var101 | Growth/Diff factor 15 | 0.42 | 1.29 | 1.20 | 1.38 |
| var121 | IGF BP2 | 53.987 | 1.08 | 1.01 | 1.16 |
| var14 | Angiopoietin-2 | 3.681 | 1.19 | 1.12 | 1.26 |
| var190 | NT pro BNP | | 1.22 | 1.16 | 1.29 |
| var95 | Glut S-Transferase alpha | 31.193 | 0.84 | 0.79 | 0.89 |
| var283 | YKL-40 | 76.692 | 1.11 | 1.05 | 1.18 |
| var49 | Chromogranin-A | 459.299 | 0.86 | 0.80 | 0.92 |
| var262 | Trefoil Factor 3 | 0.108 | 1.26 | 1.18 | 1.34 |
| var169 | MacroDerived Chemokine | 197.558 | 0.84 | 0.80 | 0.89 |
| var11 | Alpha-2-Macroglobulin | 2.678 | 1.20 | 1.13 | 1.28 |
| var232 | Selenoprotein P | 1547.56 | 0.86 | 0.82 | 0.91 |
| var247 | Tenascin-C | 227.663 | 1.14 | 1.07 | 1.21 |
| var199 | Osteoprotegerin | 2.252 | 1.21* | 1.12* | 1.31* |
| var109 | Hepat Grth Fact Receptor | 17.397 | 0.88 | 0.83 | 0.93 |
| var22 | Apolipoprotein B | 474.505 | 1.13 | 1.1 | 1.20 |
| var199*t | INTERACTION | . | | | |
| **this model included an interaction term of var199*time because the hazard for var199 violated the assumptions of non-proportionality (supremum test=0.022); the HR for var199 is based on a time period of 5 years; | | | | | |
| C statistic at max follow-up (7.8 years) = 0.64 (0.63, 0.66) to 0.76 (0.75, 0.77); NRI at this time point =0.32 (0.29, 0.36) | | | | | |

| **Sensitivity Analyses Based on Modifications of the Basic Clinical Model: Mortality** | | | | | | |
|---|---|---|---|---|---|---|
| **Parameter** | **Label** | **SD** | **Model** | **Modified Basic Clinical Model** | | |
| | | | | **Age** | **Cr**** | **Age/Cr** |
| pCV | Prior CV Event | . | 1.18 | 1.18 | 1.17 | 1.18 |
| microalb | Microalb/Alb | . | 0.97 | 1.02 | 0.97 | 1.02 |
| SVSEX | | . | 1.23 | 1.36 | 1.20 | 1.35 |
| gen_age | M>=55y/F >=65y | . | 1.37 | N/A | 1.37 | N/A |
| Age | Sensitivity Age | 7.89 | N/A | 1.29 | N/A | 1.29 |
| LDL_HDL | LDL/HDL | 1.161 | 1.09 | 1.16 | 1.09 | 1.16 |
| smk | Current Smoking | . | 1.55 | 1.68 | 1.56 | 1.68 |
| pdiab | Prior diabetes | . | 1.24 | 1.28 | 1.25 | 1.28 |
| hypten | Hypertension | . | 1.10 | 1.13 | 1.09 | 1.13 |
| Creatinine | Sensitivity Creatinine | 22.303 | N/A | N/A | 1.03 | 1.01 |
| var101 | Growth/Diff factor 15 | 0.42 | 1.29 | 1.28 | 1.28 | 1.27 |
| var121 | IGF BP2 | 53.987 | 1.08 | 1.05 | 1.08 | 1.05 |
| var14 | Angiopoietin-2 | 3.681 | 1.19 | 1.19 | 1.19 | 1.19 |
| var190 | NT pro BNP | | 1.22 | 1.21 | 1.22 | 1.21 |
| var95 | Glut S-Transferase alpha | 31.193 | 0.84 | 0.86 | 0.84 | 0.86 |
| var283 | YKL-40 | 76.692 | 1.11 | 1.14 | 1.11 | 1.15 |
| var49 | Chromogranin-A | 459.299 | 0.86 | 0.86 | 0.86 | 0.87 |
| var262 | Trefoil Factor 3 | 0.108 | 1.26 | 1.25 | 1.24 | 1.24 |
| var169 | MacroDerived Chemokine | 197.558 | 0.84 | 0.86 | 0.84 | 0.86 |
| var11 | Alpha-2-Macroglobulin | 2.678 | 1.20 | 1.16 | 1.20 | 1.16 |
| var232 | Selenoprotein P | 1547.56 | 0.86 | 0.86 | 0.86 | 0.86 |
| var247 | Tenascin-C | 227.663 | 1.14 | 1.15 | 1.14 | 1.15 |
| var199 | Osteoprotegerin | 2.252 | 1.21* | N/A | 1.22* | N/A |
| var109 | Hepat Grth Fact Receptor | 17.397 | 0.88 | N/A | 0.88 | N/A |
| var22 | Apolipoprotein B | 474.505 | 1.13 | N/A | 1.13 | N/A |
| *these models included an interaction term of age*time because the hazard for the age variable violated the assumptions of non-proportionality (supremum test <0.001); the HR for the age estimate is based on a time period of 5 years; ** this model included an interaction term of var199*time because the hazard for var199 violated the assumptions of non-proportionality (supremum test =0.04); the HR for var199 is based on a time period of 5 years | | | | | | |

| **Biomarkers for Mortality Detected with Forward Selection on 8401 Pts; HRs & C statistics & NRI Estimated Using Bootstrap Techniques** | | | | | |
|---|---|---|---|---|---|
| **Parameter** | **Label** | **SD** | **HR** | **HR ICl** | **HR uCl** |
| pCV | Prior CV Event | . | 1.17 | 1.04 | 1.32 |
| microalb | Microalb/Alb | . | 0.97 | 0.86 | 1.09 |
| SVSEX | | . | 1.23 | 1.08 | 1.42 |
| gen_age | M>=55y/F >=65y | . | 1.38 | 1.15 | 1.64 |
| LDL_HDL | LDL/HDL | 1.161 | 1.09 | 1.03 | 1.16 |
| smk | Current Smoking | . | 1.57 | 1.34 | 1.83 |
| pdiab | Prior diabetes | . | 1.23 | 1.05 | 1.44 |
| hypten | Hypertension | . | 1.11 | 0.94 | 1.29 |
| var101 | Growth/Diff factor 15 | 0.42 | 1.28 | 1.19 | 1.39 |
| var121 | IGF BP2 | 53.987 | 1.08 | 1.00 | 1.17 |
| var14 | Angiopoietin-2 | 3.681 | 1.20 | 1.13 | 1.27 |
| var190 | NT pro BNP | | 1.22 | 1.15 | 1.30 |
| var95 | Glut S-Transferase alpha | 31.193 | 0.84 | 0.80 | 0.89 |
| var283 | YKL-40 | 76.692 | 1.11 | 1.04 | 1.17 |
| var49 | Chromogranin-A | 459.299 | 0.85 | 0.79 | 0.92 |
| var262 | Trefoil Factor 3 | 0.108 | 1.26 | 1.19 | 1.33 |
| var169 | MacroDerived Chemokine | 197.558 | 0.84 | 0.80 | 0.89 |
| var11 | Alpha-2-Macroglobulin | 2.678 | 1.20 | 1.13 | 1.27 |
| var232 | Selenoprotein P | 1547.56 | 0.86 | 0.81 | 0.91 |
| var247 | Tenascin-C | 227.663 | 1.14 | 1.07 | 1.21 |
| var199 | Osteoprotegerin | 2.252 | 1.231* | 1.12* | 1.31* |
| var109 | Hepat Grth Fact Receptor | 17.397 | 0.88 | 0.83 | 0.93 |
| var22 | Apolipoprotein B | 474.505 | 1.13 | 1.0 | 1.20 |
| var199*t | INTERACTION | . | | | |
| *this model included an interaction term of var199*time because the hazard for var199 violated the assumptions of non-proportionality (supremum test=0.022); the HR for var199 is based on a time period of 5 years; C from 0.646 (0.632, 0.661) to 0.762 (0.749, 0.775); NRI 0.362 (0.284, 0.449) | | | | | |

| **Difference in Identified Biomarkers Using all 8401 vs. Model Building: Mortality** | | | |
|---|---|---|---|
| | | **Model Building (N=5630)** | **All Participants (N=8401)** |
| pCV | Prior CV Event | 1.103 | 1.18 |
| microalb | Microalb/Alb | 0.899 | 0.97 |
| SVSEX | | 1.152 | 1.23 |
| gen_age | M>=55y/F >=65y | 1.543 | 1.37 |
| LDL_HDL | LDL/HDL | 1.146 | 1.09 |
| Smk | Current Smoking | 1.683 | 1.55 |
| pdiab | Prior diabetes | 1.227 | 1.24 |
| hypten | Hypertension | 1.43* | 1.10 |
| var101 | Growth/Diff factor 15 | 1.23 | 1.29 |
| var121 | IGF BP2 | 1.13 | 1.08 |
| var14 | Angiopoietin-2 | 1.20 | 1.19 |
| var190 | NT pro BNP | 1.17* | 1.22 |
| var95 | Glut S-Transferase alpha | 0.83* | 0.84 |
| var283 | YKL-40 | 1.17 | 1.11 |
| var262 | Trefoil Factor 3 | 1.22 | 1.26 |
| var11 | Alpha-2-Macroglobulin | 1.22 | 1.20 |
| var169 | MacroDerived Chemokine | 0.84 | 0.84 |
| var207 | Peroxiredoxin-4 | 1.14 | X |
| var49 | Chromogranin-A | X | 0.86 |
| var232 | Selenoprotein P | X | 0.86 |
| var247 | Tenascin-C | X | 1.14 |
| var199 | Osteoprotegerin | X | 1.21* |
| var109 | Hepat Grth Fact Receptor | X | 0.88 |
| var22 | Apolipoprotein B | X | 1.13 |
| var199*t | INTERACTION | Yes | Yes |
| *interacting variables with time | | | |

### Example II

To explore the interaction of glargine allocation with angiopoietin 2, the following curves were constructed assessing the HR of glargine allocation for mortality according to 1 - 5 percentiles and 1-10 percentiles of angiopoietin 2 levels. No clear pattern emerges except for a suggestion that glargine may be protective in people with lower angiopoietin 2 levels.

### Figure Legend

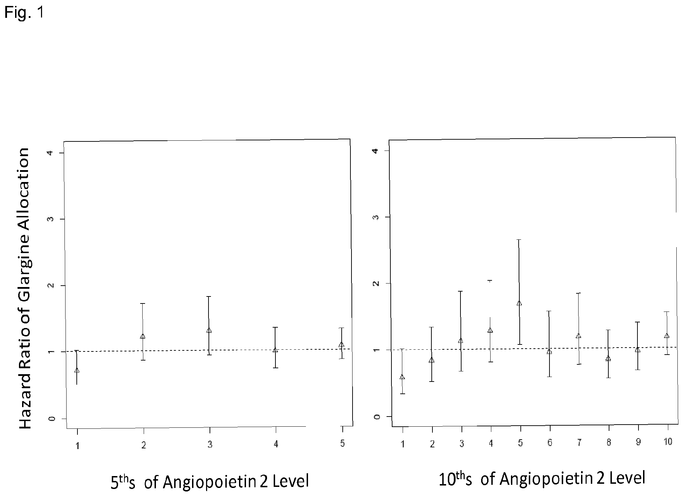

Fig. 1: Interaction of glargine allocation with angiopoietin 2; Hazard Ratio of glargine allocation for mortality according to 1 - 5 percentiles and 1-10 percentiles of angiopoietin 2 levels

### Items of the Invention

1. A method for assessing an increased risk for mortality in a subject, comprising:
   (a) determining in a sample from said subject
      (ii) the amount of at least one first marker selected from the group consisting of alpha-Glutathione-S-Transferase, Trefoil Factor 3, alpha-2-Macroglobulin, Macrophage-derived Chemokine, Apolipoprotein B, Selenoprotein P, Tenascin C and Hepatocyte Growth Factor Receptor; and
      (ii) optionally the amount of at least a further marker; and
   (b) correlating that said subject is at increased risk for mortality when said amount is altered compared to a reference amount for the at least one first marker.
2. The method according to item 1, wherein said further marker is selected from the group consisting of Nt-proBNP, Angiopoietin 2, Growth Differentiation Factor 15, Peroxiredoxin 4, YKL40, Insulin-like Growth Factor Binding Protein 2, Osteoprotegerin and Chromogranin A.
3. A method for assessing an increased risk for mortality in a subject, comprising:
   (a) determining in a sample from said subject
      (ii) the amount of at least one first marker selected from the group consisting of alpha-Glutathione-S-Transferase, Trefoil Factor 3, alpha-2-Macroglobulin, and Macrophage-derived Chemokine; and
      (ii) optionally the amount of at least a further marker; and
   (b) correlating that said subject is at increased risk for mortality when said amount is altered compared to a reference amount for the at least one first marker.
4. The method according to any one of items 1 to 3, wherein said further marker is selected from the group consisting of Nt-pro BNP, Angiopoietin-2, Growth Differentiation Factor 15, Peroxiredoxin-4, YKL-40 and Insulin-like Growth Factor Binding Protein 2.
5. The method according to according to any one of items 1 to 4, wherein said first marker is alpha-Glutathione-S-Transferase, optionally with at least one further marker.
6. The method according to any one of items 1 to 5, wherein said first and further marker is selected from the group consisting of alpha-Glutathione-S-Transferase, Nt-pro BNP and Angiopoietin-2.
7. The method according to any one of the preceding items wherein said first marker is Trefoil Factor 3, optionally with at least one further marker.
8. The method according to any one of the preceding items wherein said first marker is alpha-2-Macroglobulin, optionally with at least one further marker.
9. The method according to according to any one of the preceding items wherein said first marker is Macrophage-derived Chemokine, optionally with at least one further marker.
10. The method according to according to any one of the preceding items wherein said first marker is Apolipoprotein B, optionally with at least one further marker.
11. The method according to according to any one of the preceding items wherein said first marker is Selenoprotein P, optionally with at least one further marker.
12. The method according to according to any one of the preceding items wherein said first marker is Tenascin C, optionally with at least one further marker.
13. The method according to according to any one of the preceding items wherein said first marker is Hepatocyte Growth Factor Receptor, optionally with at least one further marker.
14. The method according to according to any one of the preceding items wherein said first marker is Hepatocyte Growth Factor Receptor and the further marker is Chromogranin A.
15. The method according to any one of items 1 to 4, wherein said first marker is selected from the group consisting of alpha-Glutathione-S-Transferase and Trefoil Factor 3.
16. The method according to any one of items 1 to 4, wherein said first marker is selected from the group consisting of alpha-Glutathione-S-Transferase and alpha-2 Macroglobulin.
17. The method according to any one of items 1 to 4, wherein said first marker is selected from the group consisting of alpha-Glutathione-S-Transferase and Macrophage-derived Chemokine.
18. The method according to any one of items 1 to 4, wherein said first marker is selected from the group consisting of Trefoil Factor 3 and alpha-2-Macroglobulin.
19. The method according to any one of items 1 to 4, wherein said first marker is selected from the group consisting of Trefoil Factor 3 and Macrophage-derived Chemokine.
20. The method according to any one of items 1 to 4, wherein said first marker is selected from the group consisting of alpha-2 Macroglobulin and Macrophage-derived Chemokine.
21. The method according to any one of items 1 to 4, wherein said first marker is selected from the group consisting of alpha-Glutathione-S-Transferase, Trefoil Factor 3, and alpha-2-Macroglobulin.
22. The method according to any one of items 1 to 4, wherein said first marker is selected from the group consisting of alpha-Glutathione-S-Transferase, Trefoil Factor 3, and Macrophage-derived Chemokine.
23. The method according to any one of items 1 to 4, wherein said first marker is selected from the group consisting of alpha-Glutathione-S-Transferase, alpha-2-Macroglobulin, and Macrophage-derived Chemokine.
24. The method according to any one of items 1 to 4, wherein said first marker is selected from the group consisting of Trefoil Factor 3, alpha-2-Macroglobulin, and Macrophage-derived Chemokine.
25. The method according to any one of items 1 to 4, wherein said first marker is Trefoil Factor 3, alpha-Macroglobulin, Macrophage-derived Chemokine and alpha-Glutathione-S-Transferase.
26. The method according to any one of items 1 to 4, wherein said first marker is alpha-Glutathione-S-Transferase, alpha-2-Macroglobulin, Macrophage-derived Chemokine, Apolipoprotein B, Selenoprotein P, Tenascin C and Hepatocyte Growth Factor Receptor.
27. The method according to any one of the preceding items wherein said first and further marker is Nt-pro BNP, alpha-Glutathione-S-Transferase, Growth Differentiation Factor 15, Trefoil Factor 3, alpha-2-Macroglobulin, Macrophage-derived Chemokine, Angiopoietin-2, YKL-40, Peroxiredoxin-4 and Insulin-like Growth Factor Binding Protein 2.
28. The method according to any one of the preceding items, wherein said first and further marker is alpha-Glutathione-S-Transferase, Trefoil Factor 3, alpha-2-Macroglobulin, Macrophage-derived Chemokine, Apolipoprotein B, Selenoprotein P, Tenascin C, Hepatocyte Growth Factor Receptor, Nt-proBNP, Angiopoietin-2, Growth Differentiation Factor 15, Peroxiredoxin-4, YKL40, Insulin-like Growth Factor Binding Protein 2, Osteoprotegerin, and Chromogranin A.
29. The method according to any one of the preceding items, wherein said further marker is selected from the group consisting of Growth Differentiation Factor 15, Insulin-like Growth Factor Binding Protein 2, Angiopoietin 2, Nt-proBNP, YKL40, Osteoprotegerin, and Chromogranin A.
30. The method according to any one of the preceding items, wherein said first marker is selected from alpha-Glutathione-S-Transferase, alpha-2-Macroglobulin, Macrophage-derived Chemokine, Apolipoprotein B, Selenoprotein P, Tenascin C and Hepatocyte Growth Factor Receptor.
31. The method according to any one of the preceding items, wherein said first and further marker is alpha-Glutathione-S-Transferase, Trefoil Factor 3, alpha-2-Macroglobulin, Macrophage-derived Chemokine, Apolipoprotein B, Selenoprotein P, Tenascin C, Hepatocyte Growth Factor Receptor, Growth Differentiation Factor 15, Insulin-like Growth Factor Binding Protein 2, Angiopoietin 2, Nt-proBNP, YKL40, Osteoprotegerin and Chromogranin A.
32. The method according to any one of the preceding items, wherein said first marker is alpha-Glutathione-S-Transferase, Trefoil Factor 3, Macrophage-derived Chemokine, alpha-2-Macroglobulin, Selenoprotein P and Tenascin C.
33. The method according to any one of the preceding items, wherein said further marker is selected from the group consisting of Growth Differentiation Factor 15, Insulin-like Growth Factor Binding Protein 2, Angiopoietin 2, Nt-proBNP, YKL40 and Chromogranin A.
34. The method according to any one of the preceding items, wherein said first marker is selected from the group consisting of alpha-Glutathione-S-Transferase, Macrophage-derived Chemokine, alpha-2-Macroglobulin, Selenoprotein P and Tenascin C.
35. The method according to any one of the preceding items, wherein said first and further marker is alpha-Glutathione-S-Transferase, Trefoil Factor 3, Macrophage-derived Chemokine, alpha-2-Macroglobulin, Selenoprotein P, Tenascin C, Growth Differentiation Factor 15, Insulin-like Growth Factor Binding Protein 2, Angiopoietin 2, Nt-proBNP, YKL40 and Chromogranin A.
36. The method according to any one of the preceding items wherein the mortality is cardiovascular mortality such as fatal myocardial infarction, fatal stroke, and/or heart failure.
37. The method according to any one of the preceding items wherein said increased risk for mortality is within the next 1-7 years.
38. The method according to any one of the preceding items wherein:
   (i) the subject is pre-diabetic or diabetic;
   (ii) the subject has an age of at least 50 years; and/or
   (iii) the subject had a previous cardiovascular disorder.
39. The method according to item 38, wherein the subject:
   (i) is pre-diabetic or diabetic; and
   (ii) has an age of at least 50 years; and
   (iii) had a previous cardiovascular disorder.
40. The method according to any one of the preceding items wherein the subject suffers from one or more of the risk factors selected from the group consisting of a previous cardiovascular disorder, albuminuria, male, age of at least 50 years, smoker, diabetic or pre-diabetic, elevated blood cholesterol levels, elevated Creatinine levels, obesity and hypertension.
41. The method of item 40, wherein the subject suffers from the risk factors previous cardiovascular disorder, albuminuria, male, age of at least 55 years, elevated blood cholesterol level, smoker, pre-diabetic or diabetic and hypertension.
42. The method according to any one of the preceding claims wherein the subject has an age of at least 55, at least 60, at least 63 or at least 65 years, preferably at least 63.
43. The method according to any one of the preceding items wherein the sample is a body fluid and/or a tissue extract.
44. The method according to item 43 wherein the body fluid is serum.
45. Use of at least one first marker selected from the group consisting of alpha-Glutathione-S-Transferase, Trefoil Factor 3, alpha-2-Macroglobulin, Macrophage-derived Chemokine, Apolipoprotein B, Selenoprotein P, Tenascin C, and Hepatocyte Growth Factor Receptor and optionally at least one further marker in the assessment of an increased risk for mortality in a subject, wherein determining an altered amount of said first marker in a sample from the subject compared to a reference amount for said marker is indicative for said risk.
46. The use according to item 45, wherein said further marker is selected from the group consisting of Nt-proBNP, Angiopoietin-2, Growth Differentiation Factor 15, Peroxiredoxin 4, YKL40, Insulin-like Growth Factor Binding Protein 2, Osteoprotegerin and Chromogranin A.
47. The use according to any one of items 45 to 46, wherein said further marker is selected from the group consisting of Nt-proBNP, Angiopoietin 2, Growth Differentiation Factor 15, YKL40, Insulin-like Growth Factor Binding Protein 2, Osteoprotegerin and Chromogranin A.
48. The use according to any one of items 45 to 47, wherein the at least one first marker is selected from the group consisting of alpha-Glutathione-S-Transferase, Trefoil Factor 3, alpha-2-Macroglobulin, and Macrophage-derived Chemokine, and optionally at least one further marker.
49. The use according to any one of items 45 to 48, wherein the at least first marker is selected from the group consisting of alpha-Glutathione-S-Transferase, Trefoil Factor 3, alpha-2-Macroglobulin, Macrophage-derived Chemokine, Selenoprotein P and Tenascin C, and optionally at least one further marker.
50. The use of at least one first marker according to any one of items 45 to 49, wherein said marker is alpha-Glutathione-S-Transferase, optionally with at least one further marker.
51. The use of at least one first marker according to any one of items 45 to 50, wherein said marker is Trefoil Factor 3, optionally with at least one further marker.
52. The use of at least one first marker according to any one of items 45 to 51, wherein said marker is alpha-2-Macroglobulin, optionally with at least one further marker.
53. The use of at least one first marker according to any one of items 45 to 52, wherein said marker is Macrophage-derived Chemokine, optionally with at least one further marker.
54. The use of at least one first marker according to any one of items 45 to 53, wherein said marker is Apolipoprotein B, optionally with at least one further marker.
55. The use of at least one first marker according to any one of items 45 to 54, wherein said marker is Selenoprotein B, optionally with at least one further marker.
56. The use of at least one first marker according to any one of items 45 to 55, wherein said marker is Tenascin C, optionally with at least one further marker.
57. The use of at least one first marker according to any one of items 45 to 56, wherein said marker is Hepatocyte Growth Factor Receptor, optionally with at least one further marker.
58. The use of at least one first marker according to any one of items 45 to 57, wherein said first marker is alpha-Glutathione-S-Transferase and Trefoil Factor 3.
59. The use of at least one first marker according to any one of items 45 to 58, wherein said first marker is alpha-Glutathione-S-Transferase and alpha-2 Macroglobulin.
60. The use of at least one first marker according to any one of items 45 to 59, wherein said first marker is alpha-Glutathione-S-Transferase and Macrophage-derived Chemokine.
61. The use of at least one first marker according to any one of items 45 to 60, wherein said first marker is Trefoil Factor 3 and alpha-2-Macroglobulin.
62. The use of at least one first marker according to any one of items 45 to 61, wherein said first marker is Trefoil Factor 3 and Macrophage-derived Chemokine.
63. The use of at least one first marker according to any one of items 45 to 62, wherein said first marker is alpha-2 Macroglobulin and Macrophage-derived Chemokine.
64. The use of at least one first marker according to any one of items 45 to 63, wherein said first marker is alpha-Glutathione-S-Transferase, Trefoil Factor 3, and alpha-2-Macroglobulin.
65. The use of at least one first marker according to any one of items 45 to 64, wherein said first marker is alpha-Glutathione-S-Transferase, Trefoil Factor 3, and Macrophage-derived Chemokine.
66. The use of at least one first marker according to any one of items 45 to 65, wherein said first marker is alpha-Glutathione-S-Transferase, alpha-2-Macroglobulin, and Macrophage-derived Chemokine.
67. The use of at least one first marker according to any one of items 45 to 66, wherein said first marker is Trefoil Factor 3, alpha-2-Macroglobulin, and Macrophage-derived Chemokine.
68. The use of at least one first marker according to any one of items 45 to 67, wherein said first marker is alpha-Glutathione-S-Transferase, alpha-2-Macroglobulin, Macrophage-derived Chemokine, Apolipoprotein B, Selenoprotein P, Tenascin C and Hepatocyte Growth Factor Receptor.
69. The use according to any one of items 45 to 68, wherein said further marker is Growth Differentiation Factor 15, Insulin-like Growth Factor Binding Protein 2, Angiopoietin 2, Nt-proBNP, YKL40 and Chromogranin A.
70. The use according to any one of items 45 to 69, wherein said first marker is alpha-Glutathione-S-Transferase, Macrophage-derived Chemokine, alpha-2-Macroglobulin, Selenoprotein P and Tenascin C.
71. Use of a marker panel, wherein said marker panel comprises alpha-Glutathione-S-Transferase, Trefoil Factor 3, alpha-2-Macroglobulin, Macrophage-derived Chemokine, Apolipoprotein B, Selenoprotein P, Tenascin C and Hepatocyte Growth Factor Receptor, and optionally at least one further marker in the assessment of an increased risk for mortality in a subject, wherein determining an altered amount of at least said marker is indicative for said risk.
72. The use according to item 71, wherein said further marker is selected from the group consisting of Nt-proBNP, Angiopoietin 2, Growth Differentiation Factor 15, Peroxiredoxin 4, YKL40, Insulin-like Growth Factor Binding Protein 2, Osteoprotegerin and Chromogranin A.
73. The use according to items 71 or 72, wherein said marker panel comprises Trefoil Factor 3, alpha-2-Macroglobulin, Macrophage-derived Chemokine, and alpha-Glutathione-S-Transferase and optionally at least one further.
74. The use according to any one of items 45 to 73, wherein said further marker is selected from the group consisting of Nt-proBNP, Angiopoietin 2, Growth Differentiation Factor 15, Peroxiredoxin 4, YKL40, and Insulin-like Growth Factor Binding Protein 2.
75. The use according to any one of items 71 to 74, wherein said marker panel comprises alpha-Glutathione-S-Transferase and Trefoil Factor 3 and optionally at least one further.
76. The use according to any one of items 71 to 75, wherein said marker panel comprises alpha-Glutathione-S-Transferase and alpha-2 Macroglobulin and optionally at least one further.
77. The use according to any one of items 71 to 76, wherein said marker panel comprises alpha-Glutathione-S-Transferase and Macrophage-derived Chemokine and optionally at least one further marker.
78. The use according to any one of items 71 to 77, wherein said marker panel comprises Trefoil Factor 3 and alpha-2-Macroglobulin and optionally at least one further marker.
79. The use according to any one of items 71 to 78, wherein said marker panel comprises Trefoil Factor 3 and Macrophage-derived Chemokine and optionally at least one further marker.
80. The use according to any one of items 71 to 79, wherein said marker panel comprises alpha-2 Macroglobulin and Macrophage-derived Chemokine and optionally at least one further.
81. The use according to any one of items 71 to 80, wherein said marker panel comprises alpha-Glutathione-S-Transferase, Trefoil Factor 3, and alpha-2-Macroglobulin and optionally at least one further.
82. The use according to any one of items 71 to 81, wherein said marker panel comprises alpha-Glutathione-S-Transferase, Trefoil Factor 3, and Macrophage-derived Chemokine and optionally at least one further marker.
83. The use according to any one of items 71 to 82, wherein said marker panel comprises alpha-Glutathione-S-Transferase, alpha-2-Macroglobulin, and Macrophage-derived Chemokine and optionally at least one further marker.
84. The use according to any one of items 71 to 83, wherein said marker panel comprises Trefoil Factor 3, alpha-2-Macroglobulin, and Macrophage-derived Chemokine and optionally at least one further marker.
85. The use according to any one of items 71 to 84, wherein said marker panel comprises alpha-Glutathione-S-Transferase, alpha-2-Macroglobulin, Macrophage-derived Chemokine, Apolipoprotein B, Selenoprotein P, Tenascin C and Hepatocyte Growth Factor Receptor and optionally at least one further marker.
86. The use according to any one of items 71 to 85, wherein said further marker is selected from the group consisting of Growth Factor Differentiation Factor 15, Insulin-like Growth Factor Binding Protein 2, Angiopoietin 2, Nt-proBNP, YKL40, Osteoprotegerin and Chromogranin A.
87. The use according to any one of items 71 to 86, wherein said marker panel comprises alpha-Glutathione-S-Transferase, Trefoil Factor 3, Macrophage-derived Chemokine, alpha-2-Macroglobulin, Selenoprotein P and Tenascin C, and optionally at least one further marker.
88. The use according to any one of items 71 to 87, wherein said marker panel comprises alpha-Glutathione-S-Transferase, Macrophage-derived Chemokine, alpha-2-Macroglobulin, Selenoprotein P and Tenascin C, and optionally at least one further marker.
89. The use according to items 71 to 88, wherein said further marker is selected from the group consisting of Growth Differentiation Factor 15, Insulin-like Growth Factor Binding Protein 2, Angiopoietin 2, Nt-proBNP, YKL40, and Chromogranin A.
90. The use according to any one of items 45-89, wherein said marker panel comprises alpha-Glutathione-S-Transferase, Angiopoietin-2 and Nt-pro BNP.
91. The use according to any one of items 71 to 90, wherein said marker panel comprises alpha-Glutathione-S-Transferase, Trefoil Factor 3, alpha-2-Macroglobulin, Macrophage-derived Chemokine, Apolipoprotein B, Selenoprotein P, Tenascin C, Hepatocyte Growth Factor Receptor, Growth Differentiation Factor 15, Insulin-like Growth Factor Binding Protein 2, Angiopoietin 2, Nt-proBNP, YKL40, Osteoprotegerin and Chromogranin A.
92. The use according to any one of items 71 to 91, wherein said marker panel comprises alpha-Glutathione-S-Transferase, Trefoil Factor 3, Macrophage-derived Chemokine, alpha-2-Macroglobulin, Selenoprotein P, Tenascin C, Growth Differentiation Factor 15, Insulin-like Growth Factor Binding Protein 2, Angiopoietin 2, Nt-proBNP, YKL40 and Chromogranin A.
93. The use according to any one of the items 71 to 90 wherein said marker panel comprises alpha-Glutathione-S-Transferase, Trefoil Factor 3, alpha-2-Macroglobulin, Macrophage-derived Chemokine, Apolipoprotein B, Selenoprotein P, Tenascin C, Hepatocyte Growth Factor Receptor, Nt-proBNP, Angiopoietin-2, Growth Differentiation Factor 15, Peroxiredoxin-4, YKL40, Insulin-like Growth Factor Binding Protein 2, Osteoprotegerin, and Chromogranin A.
94. The use according to any one of items 71 to 90, wherein said marker panel comprises alpha-Glutathione-S-Transferase, Trefoil Factor 3, alpha-2-Macroglobulin, Macrophage-derived Chemokine, Nt-proBNP, Angiopoietin 2, Growth Differentiation Factor 15, Peroxiredoxin 4, YKL40, Insulin-like Growth Factor Binding Protein 2.
95. The use according to any one of items 45-89, wherein said marker panel comprises Hepatocyte Growth Factor Receptor and Chromogranin A.
96. A kit for performing the method according to any one of items 1 to 44 comprising a reagent required to specifically determine at least alpha-Glutathione-S-Transferase, Trefoil Factor 3, alpha-2-Macroglobulin, Macrophage-derived Chemokine, Apolipoprotein B, Selenoprotein P, Tenascin C and/or Hepatocyte Growth Factor Receptor.
97. The kit according to item 96 comprising a reagent required to specifically determine at least alpha-Glutathione-S-Transferase, alpha-2-Macroglobulin, Macrophage-derived Chemokine, Apolipoprotein B, Selenoprotein P, Tenascin C and Hepatocyte Growth Factor Receptor.
98. The kit according to item 96 or 97 comprising a reagent required to specifically determine at least alpha-Glutathione-S-Transferase, Trefoil Factor 3, alpha-2-Macroglobulin, and/or Macrophage-derived Chemokine.
99. The kit according to any one of items 96 to 98 comprising a reagent required to specifically determine at least alpha-Glutathione-S-Transferase.
100. The kit according to any one of items 96-99 comprising a reagent required to specifically determine at least Trefoil Factor 3.
101. The kit according to any one of items 96 to 100 comprising a reagent required to specifically determine at least alpha-2-Macroglobulin.
102. The kit according to any one of items 96 to 101 comprising a reagent required to specifically determine at least Macrophage-derived Chemokine.
103. The kit according to any one of items 96 to 102 comprising a reagent required to specifically determine at least Apolipoprotein B.
104. The kit according to any one of items 96 to 103 comprising a reagent required to specifically determine at least Selenoprotein P.
105. The kit according to any one of items 96 to 104 comprising a reagent required to specifically determine at least Tenascin C.
106. The kit according to any one of items 96 to 105 comprising a reagent required to specifically determine at least Hepatocyte Growth Factor Receptor.
107. The kit according to any one of items 96 to 106 comprising a reagent required to specifically determine at least alpha-Glutathione-S-Transferase and Trefoil Factor 3.
108. The kit according to any one of items 96 to 107 comprising a reagent required to specifically determine at least alpha-Glutathione-S-Transferase and alpha-2 Macroglobulin.
109. The kit according to any one of items 96 to 108 comprising a reagent required to specifically determine at least alpha-Glutathione-S-Transferase and Macrophage-derived Chemokine.
110. The kit according to any one of items 96 to 109 comprising a reagent required to specifically determine at least Trefoil Factor 3 and alpha-2-Macroglobulin.
111. The kit according to any one of items 96 to 110 comprising a reagent required to specifically determine at least Trefoil Factor 3 and Macrophage-derived Chemokine.
112. The kit according to any one of items 96 to 111 comprising a reagent required to specifically determine at least alpha-2 Macroglobulin and Macrophage-derived Chemokine.
113. The kit according to any one of items 96 to 112 comprising a reagent required to specifically determine at least alpha-Glutathione-S-Transferase, Trefoil Factor 3, and alpha-2-Macroglobulin.
114. The kit according to any one of items 96 to 113 comprising a reagent required to specifically determine at least alpha-Glutathione-S-Transferase, Trefoil Factor 3, and Macrophage-derived Chemokine.
115. The kit according to any one of items 96 to 114 comprising a reagent required to specifically determine at least alpha-Glutathione-S-Transferase, alpha-2-Macroglobulin, and Macrophage-derived Chemokine.
116. The kit according to any one of items 96 to 115 comprising a reagent required to specifically determine at least alpha-Glutathione-S-Transferase, Nt-pro BNP and Angiopoietin-2.
117. The kit according to any one of items 96 to 115, comprising a reagent required to specifically determine at least Hepatocyte Growth Factor Receptor and Chromogranin A.
118. The kit according to any one of items 96 to 115 comprising a reagent required to specifically determine at least Trefoil Factor 3, alpha-2-Macroglobulin, and Macrophage-derived Chemokine.
119. The kit according to any one of items 96 to 118 comprising a reagent required to specifically determine at least alpha-Glutathione-S-Transferase, Trefoil Factor 3, Macrophage-derived Chemokine, alpha-2-Macroglobulin, Selenoprotein P and Tenascin.
120. The kit according to any one of items 92 to 119 comprising a reagent required to specifically determine at least alpha-Glutathione-S-Transferase, Macrophage-derived Chemokine, alpha-2-Macroglobulin, Selenoprotein P and Tenascin C.
121. An insulin analogue for use in reducing mortality in a subject wherein said subject expresses a reduced amount of Angiopoietin-2 compared to a reference amount.
122. An insulin analogue for use as in item 121, wherein said mortality is cardiovascular mortality such as fatal myocardial infarction, fatal stroke and/or heart failure.
123. An insulin analogue for use as in item 121 or 122, wherein said subject is pre-diabetic or diabetic.
124. An insulin analogue for use as in any one of items 121 to 123, wherein said subject is pre-diabetic or diabetic and has an age of at least 50 years.
125. An insulin analogue for use as in any one of items 121 to 123, wherein said subject is pre-diabetic or diabetic and had a previous cardiovascular disorder.
126. An insulin analogue for use as in any one of item 121 or 122, wherein said subject is pre-diabetic or diabetic, has an age of at least 50 years and had a previous cardiovascular disorder.
127. An insulin analogue for use as in any one of items 121 to 126, wherein said insulin analogue is insulin glargine.

## Claims

1. A method for assessing an increased risk for cardiovascular mortality in a subject, comprising:
(a) determining in a sample from said subject
(i) the amount of a first marker alpha-Glutathione-S-Transferase, and
(ii) the amount of the further markers Nt-pro BNP and Angiopoietin-2; and
(b) correlating that said subject is at increased risk for mortality when said amounts are altered compared to the reference amounts for the first and further markers.

2. The method according to claim 1 wherein the cardiovascular mortality is selected from fatal myocardial infarction, fatal stroke, and/or heart failure.

3. The method according to claims 1 or 2 wherein said mortality is within the next 1-7 years.

4. The method according to any one of the preceding claims wherein:
(i) the subject is pre-diabetic or diabetic;
(ii) the subject has an age of at least 50 years; and/or
(iii) the subject had a previous cardiovascular disorder.

5. The method according to claim 3 wherein the subject:
(i) is pre-diabetic or diabetic; and
(ii) has an age of at least 50 years; and
(iii) had a previous cardiovascular disorder.

6. The method according to any one of the preceding claims wherein the subject suffers from one or more of the risk factors selected from the group consisting of a previous cardiovascular disorder, albuminuria, male, age of at least 50 years, smoker, diabetic or pre-diabetic, elevated blood cholesterol levels, elevated Creatinine levels, obesity and hypertension.

7. The method of claim 6, wherein the subject suffers from the risk factors previous cardiovascular disorder, albuminuria, male, age of at least 55 years, elevated blood cholesterol level, smoker, pre-diabetic or diabetic and hypertension.

8. The method according to any one of the preceding claims wherein the sample is a body fluid, preferably serum, and/or a tissue extract.

9. Use of alpha-Glutathione-S-Transferase, Nt-pro BNP and Angiopoietin-2 in the *in vitro* assessment of the risk for development of cardiovascular mortality in a subject, wherein determining an altered amount of said markers in a sample from the subject compared to a reference amount for said markers is indicative for said risk.

10. A kit for performing the method according to any one of claims 1 to 8 comprising a reagent required to specifically determine alpha-Glutathione-S-Transferase, Nt-pro BNP and Angiopoietin-2, and optionally auxiliary reagents for performing the determination.
